# EUROPÄISCHE PATENTSCHRIFT

(11) **EP 3 129 031 B1**
(45) Veröffentlichungstag und Bekanntmachung des Hinweises auf die Patenterteilung: **15.08.2018**
(21) Anmeldenummer: 15729429.9
(22) Anmeldetag: 11.06.2015
(51) Int. Cl.: A61K 31/7028, A61K 36/28, A61K 36/534, A61K 36/232, A61K 36/53, A61P 1/00, A61K 47/36, A61K 9/06, A61K 31/726, A61K 31/728, A61K 36/23

(54) **ZUSAMMENSETZUNG ZUR BEHANDLUNG VON MAGENBESCHWERDEN**
COMPOSITION FOR TREATING STOMACH PAIN
COMPOSITION UTILISÉE POUR LE TRAITEMENT DES TROUBLES GASTRIQUES

(30) Priorität: 11.07.2014 DE 102014010244; 04.11.2014 DE 102014016158
(43) Veröffentlichungstag der Anmeldung: 15.02.2017
(73) Patentinhaber: Maria Clementine Martin Klosterfrau Vertriebsgesellschaft mbH, 50670 Köln (DE)
(72) Erfinder: UNKAUF, Markus, 80538 München (DE); VESTWEBER, Anna-Maria, 51399 Burscheid (DE)
(74) Vertreter: Von Rohr Patentanwälte Partnerschaft mbB
(86) Internationale Anmeldenummer: PCT/EP2015/063068
(87) Internationale Veröffentlichungsnummer: WO 2016/005140

(56) Entgegenhaltungen:
- WO-A1-2011/070096
- WO-A2-2010/136872
- DE-A1- 4 125 024
- US-A1- 2009 274 660

## Beschreibung

Die vorliegende Erfindung betrifft das medizinische Gebiet der Erkrankungen des Magen- bzw. Darmtrakts bzw. der Gastrointestinalerkrankungen.

Insbesondere betrifft die vorliegende Erfindung eine Zusammensetzung, insbesondere eine pharmazeutische Zusammensetzung, welche zur prophylaktischen bzw. kurativen Behandlung von Erkrankungen des Magen- bzw. Darmtrakts bzw. von Gastrointestinalerkrankungen geeignet ist.

Weiterhin betrifft die vorliegende Erfindung ein Behältnis sowie ein Arzneimittel, welche jeweils die erfindungsgemäße Zusammensetzung enthalten.

Üblicherweise werden unter dem Begriff des Verdauungstrakts diejenigen Organe des insbesondere menschlichen Körpers subsumiert, welche der Aufnahme, Zerkleinerung und dem Weitertransport der Nahrung dienen, um diese zu verdauen und die darin enthaltenen Nährstoffe dem Körper bereitzustellen. Der Verdauungstrakt setzt sich aus Mundhöhle, dem Pharynx, der Speiseröhre, dem Gastrointestinal- bzw. Magen/Darm-Trakt, der Leber sowie der Bauchspeicheldrüse zusammen. Der Gastrointestinal- bzw. Magen/Darm-Trakt wiederum besteht aus dem Magen und dem Darm.

Was den Aufbau des Magens im Speziellen anbelangt, so wird dieser durch ein Hohlorgan auf Basis eines gekrümmten muskulösen Schlauchs gebildet, welcher sich zwischen Speiseröhre (Ösophagus) und Zwölffingerdarm (Duodenum) im linken Oberbauch unterhalb des Zwerchfells befindet. Der Magen dient insbesondere der Vorverdauung von Proteinen bzw. Eiweißen. Im Gegensatz zu Proteinen passieren Kohlenhydrate und Fette den Magen ungehindert, da deren enzymatische Spaltung erst im Darm stattfindet.

Der Magen wird in die folgenden Bereiche unterteilt: Die *Pars cardiaca* bezeichnet den Mageneingang und stellt den Übergang zwischen Speiseröhre und dem Magen dar. Der sogenannte *Fundus ventriculi* bzw. Magengrund ist eine blindsackartige Erweiterung des Magens und befindet sich links und kranial vom Mageneingang. Der Magenkörper bzw. *Corpus ventriculi* macht den größten Teil des Magens aus. Auf den Magenkörper folgt der Übergang zum Dünndarm, welcher durch die *Pars pylorica,* bestehend aus Pförtnerkanal und Pförtnerhöhle, gebildet wird. Der *Pylorus* bzw. Pförtner stellt schließlich die Verbindung zum Dünndarm dar.

Der Magen bzw. die Magenwand wird durch kräftige Muskelschichten aus längs, ringförmig und schräg verlaufenden Muskelfasern gebildet, welche durch wellenförmige Bewegungen, die sogenannte Peristaltik, den Nahrungsbrei durchmischen und dem Transport der Nahrung zum Dünndarm dienen. Von innen ist die Magenwand mit einer drüsenhaltigen Magenschleimhaut (*Tunica mucosa gastrica*) ausgekleidet, welche aus einem Epithel, einer Eigenschicht (*Lamina propria mucosae*) sowie einer Verbindungsschicht zur Magenwand besteht. Die Magenschleimhaut erfüllt mehrere physiologische Funktionen:
Einerseits dient sie der Bildung von Magensäure bzw. Magensaft, welche bzw. welcher die für die im Magen stattfindende Vorverdauung erforderlichen Enzyme, insbesondere Pepsin und Kathepsin, enthält. Da die Verdauungsenzyme des Magens bei geringen pH-Werten die höchste katalytische Aktivität besitzen, liegt der pH-Wert des Magensafts bei nüchternem Magen im stark sauren Bereich von 1 bis 1,5. Durch das saure Milieu im Magen werden zudem die meisten in der Nahrung enthaltenen Bakterien abgetötet, so dass dem Magen auch eine gewisse Funktion als Infektionsschutz zukommt.

Andererseits wird durch spezielle Mukosazellen der Magenschleimhaut eine circa 0,5 mm dicke Schleimschicht insbesondere auf Basis von Proteoglykanen ausgebildet, welche das außen liegende Muskelgewebe des Magens sowie die Magenschleimhaut vor der Selbstverdauung durch den sauren Magensaft schützt. Darüber hinaus sondern die Zellen Hydrogencarbonat ab, welches ebenfalls dem Schutz des Magens dient, da es sich in der Schleimschicht sammelt und so die starke Säure des Magensafts (d. h. Salzsäure) im Bereich der Magenwand bzw. der Magenschleimhaut abschwächt.

Grundsätzlich funktioniert das physiologische System des Magens einwandfrei. In der modernen Gesellschaft leiden jedoch zunehmend mehr Menschen an Beschwerden des Magen/Darm-Trakts, wobei die Ursachen äußerst vielfältig sind. Häufige Ursachen für derartige Beschwerden sind Bewegungsmangel, Stress, Nahrungsmittel- und Genussmittelunverträglichkeiten, aber auch Krankheiten und Medikamente, welche zu einer Schädigung der Magenschleimhaut führen. Darüber hinaus können auch Infektionen mit dem Bakterium *Helicobacter pylori* zu verschiedensten Magenproblemen, wie Sodbrennen, Gastritis oder Magengeschwüren, führen. Bei langanhaltenden Magenbeschwerden kann sogar das Risiko, an einem Magenkarzinom zu erkranken, erhöht sein.

Ein Großteil von Magenbeschwerden verschiedenster Art, wie Sodbrennen, Gastritis, Magengeschwüren, Magendruck, Reizmagen oder Hyperazidität, geht mit einer krankhaften Veränderung bzw. Schädigung der Magenschleimhaut einher. Dabei wird der Schutzfilm aus Schleim, welcher den Magen vor der Magensäure schützt, durchlässig. Die eigentlich der Verdauung insbesondere von Peptiden dienende Magensäure greift dann aufgrund des zumindest teilweise fehlenden Schutzfilms die oberflächlichen Schleimhautschichten an und führt zu schmerzhaften Entzündungen der Magenwand, welche auch als Gastritis bezeichnet werden. Eine solche akute Gastritis kann bei Nichterkennen bzw. Nichtbehandlung in eine chronische Gastritis übergehen bzw. in der Ausbildung von Magengeschwüren (Ulcus) resultieren. Bei Magengeschwüren liegt nicht mehr eine ausschließlich oberflächliche Schädigung der Magenschleimhaut vor, sondern es entsteht vielmehr ein Geschwür, welches mit der Zerstörung von Gewebe einhergeht. Dabei kann der Gewebeverlust bis in tiefe Schichten der Magenschleimhaut hineinreichen.

Je nach Schwere und Ursache der Erkrankungen bzw. Beschwerden des Magen- und Darmtrakts kommen unterschiedliche Medikamente zum Einsatz: In leichteren Fällen genügt die Neutralisierung der Magensäure mit sogenannten Antazida, beispielsweise auf Basis von Hydroxiden, Carbonaten und Silikaten des Aluminiums, Magnesiums, Calciums und Natriums (z. B. Aluminiumhydroxid, Magnesiumhydroxid, Magnesiumtrisilikat, Calciumcarbonat, Natriumbicarbonat etc.).

Antazida, insbesondere aluminiumhaltige Antazida weisen jedoch oftmals unangenehme Nebenwirkungen auf. Insbesondere kann es zu Obstipationen kommen. Darüber hinaus kann die Einnahme größerer Mengen aluminiumbasierter Antazida zu Aluminium-Intoxikationen (z. B. Dialyse-Enzephalopathie) führen. Weiterhin kommt es häufig zu unerwünschten Wechselwirkungen mit anderen Arzneimitteln, wie Tetracyclinen, Digoxin, Chinolonen etc. Aluminiumhaltige Zusammensetzungen dieser Art werden beispielsweise in der EP 2 435 021 B1 beschrieben. Weiterhin können calciumbasierte Antazida zu einer sogenannten Hypercalcämie, zu einer Alkalose und in schweren Fällen auch zu Niereninsuffizienz (Milch-Alkali-Syndrom) führen. Natriumbicarbonat als Antazidum wiederum kann beispielsweise zu einer metabolischen Alkalose führen; auch ist die Natriumbelastung nicht zu vernachlässigen, und außerdem führt die CO₂-Bildung zu einem unerwünschten Aufstoßen und kann sogar zu einer Magenruptur führen.

Zwar führen die vorgenannten Antazida zu einer Neutralisierung der Magensäure, so dass zumindest kurzfristig eine Linderung der Magenreizung eintritt, jedoch wird kein zusätzlicher Schutz für das angegriffene Gewebe bereitgestellt. Mittelfristig sinkt jedoch der pH-Wert im Magen wieder, so dass erneute Magen- bzw. Schleimhautreizungen und Entzündungssymptome auftreten. Darüber hinaus ist ihre Einnahme teilweise mit zahlreichen Nebenwirkungen der vorgenannten Art verbunden.

Die US 2009/0274660 A1 betrifft Zusammensetzungen auf Basis von Glykosaminoglykanen, polaren oberflächenaktiven Lipiden, L-Aminosäuren und Glycin sowie verschiedenen Zusatzstoffen zur Behandlung der entzündlichen Symptome von Morbus Crohn, insbesondere im Hinblick auf die Gewebeheilung und Geweberegeneration.

Weiterhin betrifft die WO 2011/070096 A1 eine Zusammensetzung zur Behandlung von Magen/Darm-Beschwerden, wobei die Zusammensetzung Auszüge, Tinkturen und/oder Extrakte von Pflanzen bzw. Pflanzenteilen von *Chamomilla recutica, Melissa officinalis, Mentha x piperita, Carum carvi* und *Centaurium erythraea* enthält.

Die DE 41 25 024 A1 betrifft ein Arzneimittel auf pflanzlicher Basis mit tonisierender Wirkung auf glatte, muskuläre Organe, wie Magen und Darm. Die darin beschriebene Zusammensetzung enthält Frischpflanzenauszüge aus *Iberis amara totalis.* Darüber hinaus wird auch der Einsatz von Bestandteilen aus Schleifenblume, Kamille, Kümmel, Melisse und Süßholzwurzel beschrieben.

Zudem betrifft die WO 2010/136872 A2 die Verwendung einer Kombination von Hyaluronsäure, Chondroitinsulfat und Aluminiumhydroxid zur Herstellung von Medikamenten für die Behandlung von gastrointestinalen Beschwerden, insbesondere zur Behandlung von Gastritis oder Schädigungen des Epithels im Magen bzw. Darm.

Der vorliegenden Erfindung liegt daher die Aufgabe zugrunde, eine Zusammensetzung bzw. ein Arzneimittel bereitzustellen, welche bzw. welches sich zur prophylaktischen bzw. kurativen Behandlung von Erkrankungen des Magen/Darm-Trakts bzw. zur Behandlung von Gastrointestinalerkrankungen eignet, wobei die zuvor geschilderten Nachteile des Standes der Technik zumindest weitgehend vermieden oder aber wenigstens abgeschwächt werden sollen.

Insbesondere liegt der vorliegenden Erfindung die Aufgabe zugrunde, eine Zusammensetzung bzw. ein Arzneimittel bereitzustellen, welche bzw. welches sich einerseits durch eine hohe sowie lang anhaltende Wirkeffizienz in Bezug auf die Linderung von Beschwerden des Magen/Darm-Trakts und andererseits durch eine gute Verträglichkeit auszeichnet und zumindest im Wesentlichen nebenwirkungsfrei ist. Darüber hinaus soll die Zusammensetzung einen verbesserten Schutz des Magens, insbesondere der Magenschleimhaut und der Magenwand, vor Magensaft bzw. Magensäure gewährleisten.

Zur Lösung der zuvor geschilderten Aufgabe schlägt die vorliegende Erfindung eine Zusammensetzung gemäß Anspruch 1 vor; weitere vorteilhafte Ausgestaltungen sind Gegenstand der diesbezüglich abhängigen Ansprüche.

Weiterer Gegenstand der vorliegenden Erfindung ist ein Arzneimittel gemäß dem diesbezüglich unabhängigen Anspruch, welches die erfindungsgemäße Zusammensetzung aufweist und für die erfindungsgemäße Verwendung geeignet ist.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoff oder dergleichen stets 100% bzw. 100 Gew.-% resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Gegenstand der vorliegenden Erfindung ist somit - gemäß einem **ersten** Aspekt der vorliegenden Erfindung - eine Zusammensetzung, insbesondere pharmazeutische Zusammensetzung, zur prophylaktischen und/oder kurativen Behandlung von Erkrankungen des Magen- und/oder Darmtrakts und/oder von Gastrointestinalerkrankungen, wobei die Zusammensetzung - in Kombination und in jeweils wirksamen, insbesondere pharmazeutisch wirksamen Mengen -
(a) Hyaluronsäure oder deren Salze ("Komponente (a)") in einer relativen Menge im Bereich von 0,2 bis 5 Gew.-%, bezogen auf die Zusammensetzung, und
(b) eine Kombination von
   Kamillenblüten-Extrakt ("Komponente (b1)") in einer relativen Menge, bezogen auf die Zusammensetzung, im Bereich von 10 bis 40 Gew.-%, Kümmelfrüchte-Extrakt ("Komponente (b2)") in einer relativen Menge, bezogen auf die Zusammensetzung, im Bereich von 2 bis 20 Gew.-%, Melissenblätter-Extrakt ("Komponente (b3)") in einer relativen Menge, bezogen auf die Zusammensetzung, im Bereich von 2 bis 20 Gew.-%, und Pfefferminzblätter-Extrakt ("Komponente (b4)") in einer relativen Menge, bezogen auf die Zusammensetzung, im Bereich von 1 bis 10 Gew.-%,
enthält.

Der Begriff "pharmazeutische Zusammensetzung" bzw. "pharmazeutische Zubereitung", wie er im Rahmen der vorliegenden Erfindung verwendet wird, ist sehr breit zu verstehen und umfasst nicht nur pharmazeutische Präparate bzw. Pharmazeutika und Arzneimittel als solche, sondern auch sogenannte Medizinprodukte, Lebensmittel oder Nahrungsergänzungsmittel.

Im Rahmen der vorliegenden Erfindung wird auf Basis der erfindungsgemäßen Kombination von Hyaluronsäure einerseits und zielgerichtet und speziell ausgewählten Pflanzenextrakten der zuvor definierten Art andererseits erstmals eine Zusammensetzung bereitgestellt, welche die Symptome von Magenbeschwerden, insbesondere Magenschmerzen, Sodbrennen, Reizmagen, Gastritis sowie Schleimhautschädigungen, mit äußerst hoher Wirkeffizienz lindert, gleichzeitig jedoch auch eine hervorragende Verträglichkeit aufweist und einen langanhaltenden Schutz der Magenschleimhaut bzw. des Magens gewährleistet (und zwar ohne die Anwesenheit von Antazida bzw. neutralisierenden Verbindungen).

Was die Komponente (a) der erfindungsgemäßen Zusammensetzung anbelangt, so ist diesbezüglich Folgendes auszuführen.

Bei den erfindungsgemäß verwendeten vorzugsweise sauren Glykosaminoglykanen ("Komponente (a)") handelt es sich um lineare Mucopolysaccharide, welche aus sich wiederholenden Sacchariden bzw. Saccharid-Einheiten gebildet werden. Im Allgemeinen bestehen die Saccharid-Einheiten aus einer Uronsäure, wie insbesondere Glucuronsäure, aber auch Iduronsäure oder Uronsäure, welche 1,3-glykosidisch mit einem Aminozucker, wie N-Acetylglucosamin, verknüpft sind. Die Saccharid-Einheiten zur Ausbildung der Kette als solcher wiederum sind 1,4-glykosidisch miteinander verbunden. Insbesondere kann es auch vorgesehen sein, dass die Glykosaminoglykane mit Schwefelsäure oder Essigsäure weiter verestert sind. Die in Rede stehenden Glykosaminoglykane liegen häufig in Assoziation mit biologischen Makromolekülen vor und können beispielsweise in großer Zahl kovalent an Proteine gebunden vorkommen. Auf diese Weise können sie das Gerüst vieler faserbildender Stoffe, wie z.B. Fibrillin, im Körper ausbilden. Ein erfindungsrelevanter Vorteil bzw. eine erfindungsrelevante Eigenschaft der Glykosaminoglykane liegt insbesondere in ihrer Fähigkeit, in großen Mengen Wasser aufnehmen zu können, so dass bei oraler Einnahme eine Art Muzinschicht auf der Magenschleimhaut ausgebildet wird.

Eine besonders gute Wirkung wird im Rahmen der vorliegenden Erfindung erzielt, wenn als Glykosaminoglykan Hyaluronsäure oder deren Salze eingesetzt wird. Chemisch gesehen handelt es sich bei Hyaluronsäure um eine makromolekulare Kette aus Disacchariden auf Basis von D-Glucuronsäure und N-Acetyl-D-Glucosamin, welche über eine beta-1,3-glykosidische Bindung miteinander verknüpft sind. Die Disaccharide wiederum sind über eine beta-1,4-glykosidische Bindung zu einer polymeren Kette miteinander verbunden. Im Allgemeinen besteht ein Hyaluronsäure-Polymer aus 250 bis 50.000 oder mehr Disaccharideinheiten.

Hyaluronsäure stellt im menschlichen bzw. tierischen Körper einen wichtigen Bestandteil des Bindegewebes dar und spielt darüber hinaus eine Rolle bei der Zellproliferation und Zellmigration. Weiterhin besitzt auch Hyaluronsäure die Fähigkeit, äußerst große Mengen an Wasser zu binden: So können mit 1 g Hyaluronsäure bis zu 6 Liter Wasser gebunden werden.

Durch die Bindung großer Mengen an Wasser kann im Rahmen der vorliegenden Erfindung überraschenderweise in effizienter Weise eine Schleimschicht auf der Magenschleimhaut ausgebildet werden. Darüber hinaus unterstützt die Hyaluronsäure die Regeneration bzw. Heilung der geschädigten Magenschleimhaut und Magenwand.

Neben der physiologischen bzw. pharmakologischen Wirkung kann darüber hinaus die Konsistenz bzw. die Viskosität bzw. die Rheologie der erfindungsgemäßen über den Einsatz der sauren Glykosaminoglykone, insbesondere der Hyaluronsäure, gesteuert werden, so dass grundsätzlich auf den Einsatz weiterer Rheologiemodifikatoren bzw. Gelbildner verzichtet werden kann.

Was die erfindungsgemäß eingesetzte Komponente (b) der Zusammensetzung nach der vorliegenden Erfindung anbelangt, ist diesbezüglich Folgendes auszuführen:
Bei der Komponente (b) handelt es sich um eine Kombination von Kamillenblüten-Extrakt, Kümmelfrüchte-Extrakt, Melissenblätter-Extrakt und Pfefferminzblätter-Extrakt sowie gegebenenfalls Angelikawurzel-Extrakt.

Bei dem erfindungsgemäß eingesetzten Angelikawurzel-Extrakt handelt es sich insbesondere um den Auszug aus der Arznei-Engelwurz bzw. der echten Engelwurz (*Angelica archangelica*), welche der Familie der Doldenblütler angehört. Angelikawurzel-Extrakt enthält als pharmakologisch bedeutsame Inhaltsstoffe vorwiegend Monoterpene, insbesondere alpha- und beta-Phellandren und alpha-Pinen. Darüber hinaus weist Angelikawurzel-Extrakt - in deutlich geringeren Mengen jedoch - Sesquiterpene, rund 20 photosensibilisierende Furanocumarine sowie Angelika- und Fumarsäure, Chlorogen- und Kaffeesäure, Harze und Flavanone auf. Die vorgenannten Inhaltsstoffe begründen die karminative und antimikrobielle Wirkung von Angelikawurzel-Extrakt. Darüber hinaus wird durch die Inhaltsstoffe des Extrakts die Produktion von Magensaft und Bauchspeicheldrüsensekret angeregt. Für weitergehende Einzelheiten zu Angelikawurzel bzw. Angelikawurzel-Extrakt kann insbesondere verwiesen werden auf RÖMPP Chemielexikon, 10. Auflage, Georg Thieme-Verlag, Stuttgart/New York, Band 1, 1997, Seite 193, Stichwort: "Angelikawurzel", sowie die darin jeweils referierte Literatur, deren gesamter Inhalt hiermit durch Bezugnahme eingeschlossen ist. Kamillenblüten-Extrakt, wie er erfindungsgemäß eingesetzt wird, wird insbesondere aus den Blütenständen der echten Kamille (*Matricaria chamomilla*) gewonnen, welche der Familie der Korbblütler angehört. Kamillenblüten-Extrakt enthält als pharmakologisch wirksame Inhaltsstoffe Substanzen aus der Gruppe der Sesquiterpene, Flavonoide und Cumarine, wobei Chamazulen sowie das Sesquiterpen Bisabolol die Hauptwirkstoffe sind. Die Inhaltsstoffe von Kamille bzw. Kamillenblüten-Extrakt wirken antiphlogistisch bzw. entzündungshemmend und darüber hinaus krampflösend. Für weitergehende Einzelheiten zu Kamillenblüten-Extrakt bzw. Kamille kann verwiesen werden auf RÖMPP Chemielexikon, 10. Auflage, Georg Thieme-Verlag, Stuttgart/New York, Band 3, 1997, Seite 2075, Stichwort: "Kamille" und Stichwort: "Kamillenöl", sowie die dort jeweils referierte Literatur, deren gesamter Inhalt hiermit durch Bezugnahme eingeschlossen ist.

Was den erfindungsgemäß eingesetzten Kümmelfrüchte-Extrakt anbelangt, so kann dieser beispielsweise durch Extraktion, insbesondere der Samen, der Kümmelpflanze, botanisch *Carum carvi,* gewonnen werden. Kümmelfrüchte-Extrakt enthält als Hauptinhaltsstoffe insbesondere Carvon und Limonen. Weitere Inhaltsstoffe von Kümmelfrüchte-Extrakt sind Myrcen, alpha-Phellandren, beta-Cymol, beta-Carophyllen, cis- bzw. trans-Carveol, cis- bzw. trans-Dihydrocarvon, trans-Dihydrocarveol sowie alpha- und/oder beta-Pinen. Die vorgenannten Inhaltsstoffe begründen die karminative Wirkung des Kümmelfrüchte-Extrakts bei dyspeptischen Beschwerden, insbesondere bei Völlegefühl und Blähungen. Neben der spasmolytischen Wirkung an der glatten Muskulatur des Magen/Darm-Trakts besitzt Kümmelöl darüber hinaus auch antimikrobielle Eigenschaften. Für weitergehende Einzelheiten zu Kümmel bzw. Kümmelfrüchte-Extrakt kann verwiesen werden auf RÖMPP Chemielexikon, 10. Auflage, Georg Thieme Verlag, Stuttgart/New York, Band 3, 1997, Seite 2295, Stichwort: "Kümmel" und Stichwort: "Kümmelöl", sowie die dort jeweils referierte Literatur, deren gesamter Inhalt hiermit durch Bezugnahme eingeschlossen ist.

Bei dem erfindungsgemäß eingesetzten Melissenblätter-Extrakt handelt es sich insbesondere um den Extrakt aus Zitronenmelisse bzw. Melisse (*Melissa officinalis*), welche der Familie der Lippenblütler angehört. Die pharmakologisch bedeutsamen Inhaltsstoffe aus Melissenblättern sind insbesondere Hydroxyzimtsäure-Derivate, insbesondere Rosmarinsäure und Kaffeesäure, Chlorogensäure sowie ätherische Öle. Die wichtigsten Komponenten der ätherischen Öle sind die Citrale, insbesondere Geranial und Neral, Citroneral sowie beta-Caryophyllen. Darüber hinaus enthält Melissenblätter-Extrakt Bitterstoffe, Harze, Schleimstoffe, Glykoside, Saponine und Thymol. Die pharmakologische Wirkung von Melissenblätter-Extrakt beruht auf beruhigenden sowie krampflösenden und antibakteriellen Eigenschaften. Für weitergehende Einzelheiten zu Melissenblätter-Extrakt bzw. Melisse kann verwiesen werden auf RÖMPP Chemielexikon, 10. Auflage, Georg Thieme-Verlag, Stuttgart/New York, Band 4, 1997, Seite 2580, Stichwort: "Melissenöl" sowie die dort jeweils referierte Literatur, deren gesamter Inhalt hiermit durch Bezugnahme eingeschlossen ist.

Der erfindungsgemäß eingesetzte Pfefferminzblätter-Extrakt wird insbesondere aus den oberirdischen Teilen der Pfefferminze, botanisch *Mentha piperita,* insbesondere aus deren Blättern, gewonnen. Erfindungsgemäß eingesetzter Pfefferminzblätter-Extrakt kann insbesondere Menthol und/oder Menthylacetat enthalten. Pfefferminzblätter-Extrakt besitzt bezüglich seiner pharmakologischen Relevanz eine spasmolytische Wirkung an der Muskulatur des Magen/Darm-Trakts und wirkt darüber hinaus karminativ. Für weitergehende Einzelheiten zu dem erfindungsgemäß eingesetzten Pfefferminzöl kann verwiesen werden auf RÖMPP Lexikon Naturstoffe, erste Auflage, Georg Thieme Verlag, Stuttgart/New York, 1997, Seiten 478 und 479, Stichwort: "Pfefferminzöl" und die dort referierte Literatur, deren gesamter Inhalt hiermit durch Bezugnahme eingeschlossen ist.

Die Anmelderin hat im Rahmen der vorliegenden Erfindung überraschend gefunden, dass sich Hyaluronsäure einerseits und Pflanzenextrakt(e), ausgewählt aus Angelikawurzel-Extrakt, Kamillenblüten-Extrakt, Kümmelfrüchte-Extrakt, Melissenblätter-Extrakt bzw. Pfefferminzblätter-Extrakt, andererseits bezüglich ihrer Wirkung bei der Behandlung von Beschwerden des Gastrointestinaltrakts synergistisch ergänzen. Durch die jeweilige Wirkweise der Einzelkomponenten erfolgt nämlich eine Behandlung der Beschwerden auf mehreren Ebenen, wie nachfolgend im Detail geschildert.

Die Wirkweise der erfindungsgemäßen Zusammensetzung kann dabei - ohne sich hierbei jedoch auf diese Theorie beschränken zu wollen - wie folgt beschrieben werden:
Bei Einsatz der erfindungsgemäßen Zusammensetzung zur Behandlung von Beschwerden des Gastrointestinaltrakts bilden die in der Zusammensetzung enthaltene Hyaluronsäure bzw. deren Salze aufgrund ihrer Eigenschaft, große Mengen Wasser binden zu können, eine Art Matrix aus und lagern sich - ähnlich wie die von der Magenschleimhaut produzierten Proteoglykane - als Muzinschicht an der Magenschleimhaut an, so dass ein Wiederaufbau bzw. eine Reparatur der teilweise fehlenden bzw. geschädigten Muzinschicht erfolgt. Auf dieser Basis gewährleistet die Zusammensetzung nach der vorliegenden Erfindung einen langanhaltenden Schutz der angegriffenen Magenschleimhaut bzw. Magenwand vor dem stark sauren Magensaft. Zudem unterstützen die Hyaluronsäure bzw. deren Salze die Regeneration bzw. Heilung der angegriffenen Magenschleimhaut bzw. Magenwand. Die speziell und zielgerichtet ausgewählten Pflanzenextrakte wiederum entfalten im Magen insbesondere karminative, krampflösende und antiphlogistische Eigenschaften, was eine Linderung der Schmerz- und Entzündungssymptomatik bewirkt und darüber hinaus funktionelle und motilitätsbedinge Störungen bzw. Beschwerden des Magen- und Darmtrakts bessert.

Auf Basis der vorliegenden Erfindung wird somit erstmals eine pharmazeutische Zusammensetzung bereitgestellt, welche auch über einen langen Zeitraum einen Schutz des Magens bzw. der Magenschleimhaut der Erkrankungen bzw. Fehlfunktionen des Magen-Darm-Trakts gewährleistet (und zwar ohne die Nebenwirkungen herkömmlicher, für diesen Zweck eingesetzter Pharmazeutika).

Darüber hinaus zeichnet sich die erfindungsgemäße Zusammensetzung durch ihre hervorragende Verträglichkeit aus und ist zumindest im Wesentlichen frei von unerwünschten Nebenwirkungen. Insbesondere treten zumindest im Wesentlichen keine Kreuzreaktionen mit anderen Medikamenten oder Arzneimitteln auf. Darüber hinaus ist die erfindungsgemäße Zusammensetzung frei von Nebenwirkungen, wie sie üblicherweise mit aluminium- oder calciumhaltigen oder anderweitigen Antazida einhergehen.

Die erfindungsgemäße Zusammensetzung bewirkt zudem durch die eingesetzte Hyaluronsäure bzw. deren Salze einerseits sowie die speziell ausgewählten Pflanzenextrakte andererseits eine beschleunigte Regeneration der angegriffenen Magenschleimhaut bzw. Magenwand, was insbesondere auch - ohne sich hierbei auf diese Theorie zu beschränken - auf die physiologische Relevanz von Hyaluronsäure bei der Zellproliferation zurückzuführen ist.

Auch werden durch die erfindungsgemäße Zusammensetzung motilitätsbedingte Störungen des Gastrointestinaltrakts gelindert, da die im Rahmen der erfindungsgemäßen Zusammensetzung eingesetzten pflanzlichen Extrakte durch ihre krampflösenden und karminativen Eigenschaften die peristaltischen Bewegungen des Magen/Darm-Trakts positiv stimulieren und eine Entspannung der verkrampfen Muskulatur bewirken, was wiederum in einer Schmerzlinderung resultiert.

Schließlich erfolgt auf Basis der eingesetzten Hyaluronsäure bzw. deren Salzen sowie der spezifisch ausgewählten Pflanzenextrakte eine hochwirksame Linderung von Entzündungsreaktionen, so dass insbesondere Beschwerden, wie akute oder chronische Gastritis, auf Basis der erfindungsgemäßen Zusammensetzung wirkungsvoll behandelt werden können.

Insgesamt zeichnet sich die erfindungsgemäße Zusammensetzung gegenüber den aus dem Stand der Technik bekannten Zusammensetzungen zur Behandlung von Gastrointestinalerkrankungen bzw. Beschwerden des Magen/Darm-Trakts somit durch ihre äußerst hohe Wirkeffizienz in Bezug auf einen Schutz der Magenschleimhaut bzw. der Magenwand vor Magensäure und eine Linderung der Schmerz- und Entzündungssymptomatik einerseits sowie durch ihre signifikant verbesserte Verträglichkeit andererseits aus.

Die erfindungsgemäßen Zusammensetzungen können in vielfältiger Art und Weise ausgestaltet sein. Bevorzugte Ausführungsformen werden nachfolgend im Detail erläutert:
In Bezug auf die erfindungsgemäß eingesetzten Pflanzenextrakte ist es vorgesehen, dass die Zusammensetzung eine Kombination von vier voneinander verschiedenen Pflanzenextrakten, bevorzugt von fünf voneinander verschiedenen Pflanzenextrakten, wie sie zuvor definiert wurden, aufweist. Die signifikante Wirksamkeitssteigerung durch den Einsatz mehrerer Pflanzenextrakte ist vermutlich - ohne sich hierbei auf diese Theorie beschränken zu wollen - darauf zurückzuführen, dass die spezifisch ausgewählten Pflanzenextrakte jeweils eine individuelle Zusammensetzung an Inhalts- bzw. Wirkstoffen, insbesondere auf Basis von verschiedensten sekundären Pflanzenstoffen, besitzen, welche sich in ihren Wirkungen gegenseitig ergänzen, so dass durch den Einsatz mehrerer Extrakte in Kombination ein deutlich breiteres Wirkungsspektrum erreicht wird.

Erfindungsgemäß weist die Zusammensetzung als Komponente (b) eine Kombination von Kamillenblüten-Extrakt ("Komponente (b1)"), Kümmelfrüchte-Extrakt ("Komponente (b2)"), Melissenblätter-Extrakt ("Komponente (b3)") und Pfefferminzblätter-Extrakt ("Komponente (b4)") auf.

Gemäß einer weiteren, gleichermaßen besonders bevorzugten Ausführungsform der vorliegenden Erfindung weist die Zusammensetzung als Komponente (b) eine Kombination von Kamillenblüten-Extrakt ("Komponente (b1)"), Kümmelfrüchte-Extrakt ("Komponente (b2)"), Melissenblätter-Extrakt ("Komponente (b3)"), Pfefferminzblätter-Extrakt ("Komponente (b4)") und Angelikawurzel-Extrakt ("Komponente (b5)") auf.

Gemäß einer bevorzugten Ausführungsform der vorliegenden Erfindung ist es somit vorgesehen, dass die erfindungsgemäße Zusammensetzung, insbesondere pharmazeutische Zusammensetzung, welche sich vorzugsweise zur prophylaktischen und/oder kurativen Behandlung von Erkrankungen des Magen- und/oder Darmtrakts und/oder von Gastrointestinalerkrankungen eignet, insbesondere eine wie zuvor beschriebene Zusammensetzung, - in Kombination und in jeweils wirksamen, insbesondere pharmazeutisch wirksamen Mengen -
(a) Hyaluronsäure oder deren Salze sowie
(b) eine Kombination der folgenden Pflanzenextrakte:
   (b1) Kamillenblüten-Extrakt;
   (b2) Kümmelfrüchte-Extrakt;
   (b3) Melissenblätter-Extrakt;
   (b4) Pfefferminzblätter-Extrakt;
   gegebenenfalls (b5) Angelikawurzel-Extrakt;
enthält.

Die Mengen der jeweiligen Inhaltsstoffe bzw. Komponenten der erfindungsgemäßen Zusammensetzung können in weiten Bereichen variieren. Es versteht sich von selbst, dass bei den nachfolgenden Mengenangaben im Fall von Prozentangaben die Inhaltsstoffe so zu kombinieren sind, dass die Prozentangaben (gegebenenfalls unter Einbezug weiterer Inhaltsstoffe) insgesamt stets 100 % bzw. 100 Gew.-% ergeben. Im Übrigen gilt, dass der Fachmann anwendungsbezogen oder einzelfallbedingt von den nachfolgend aufgeführten Mengenangaben abweichen kann, ohne dass er den Rahmen der vorliegenden Erfindung verlässt.

In Bezug auf den Einsatz der Hyaluronsäure bzw. deren Salzen ist es vorgesehen, dass die Zusammensetzung diese(s) bzw. die Komponente (a) in einer relativen Menge im Bereich von 0,2 bis 5 Gew.-%, besonders bevorzugt im Bereich von 0,3 bis 2,5 Gew.-%, noch mehr bevorzugt 0,5 bis 1,5 Gew.-%, bezogen auf die Zusammensetzung, enthält.

Darüber hinaus ist es im Rahmen der vorliegenden Erfindung vorgesehen, dass die Zusammensetzung die Hyaluronsäure bzw. deren Salze bzw. die Komponente (a) in einer absoluten Menge im Bereich von 0,01 bis 25 mg, insbesondere im Bereich von 0,1 bis 15 mg, vorzugsweise im Bereich von 0,5 bis 2,5 mg, noch mehr bevorzugt im Bereich von 0,6 bis 2 mg, enthält, bezogen auf eine Applikationseinheit der Zusammensetzung.

Was den Begriff "Applikationseinheit" anbelangt, so ist dieser im Rahmen der vorliegenden Erfindung insbesondere derart zu verstehen, dass er auf die Wirkstoffmenge der Zusammensetzung bezogen ist, welche im Rahmen einer einzelnen Applikation (Einzelapplikation) bzw. einer einzelnen Dosis (Einzeldosis) an den Patienten verabreicht wird.

Im Zusammenhang mit dem Schutz der Magenschleimhaut bzw. des Magens vor Magensäure sowie die Förderung der Regeneration der Magenschleimhaut hat es sich als besonders wirkungsvoll erwiesen, wenn die Komponente (a) Hyaluronsäure oder deren physiologisch unbedenkliche Salze, vorzugsweise das Natriumsalz der Hyaluronsäure, ist.

Darüber hinaus hat es sich als vorteilhaft in Bezug auf die Wirksamkeit erwiesen, wenn die Hyaluronsäure ein definiertes Molekulargewicht aufweist. Besonders gute Ergebnisse bei der Behandlung von Beschwerden des Gastrointestinaltrakts werden erzielt, wenn die Komponente (a), insbesondere die Hyaluronsäure oder deren physiologisch unbedenkliche Salze, ein Molekulargewicht, insbesondere ein zahlenmittleres Molekulargewicht, im Bereich von 1 bis 100.000 kDa (Kilodalton), insbesondere im Bereich von 10 bis 50.000 kDa, vorzugsweise im Bereich von 100 bis 10.000 kDa, bevorzugt im Bereich von 750 bis 5.000 kDa, besonders bevorzugt im Bereich von 900 bis 2.000 kDa, aufweist.

Was die eingesetzte Menge an Pflanzenextrakt(en) bzw. Komponente (b) anbelangt, so kann diese ebenfalls in weiten Bereichen variieren:

Besonders gute Ergebnisse werden erzielt, wenn die Zusammensetzung Pflanzenextrakt(e) bzw. die Komponente (b) in einer relativen Gesamtmenge, bezogen die Zusammensetzung, im Bereich von 1 bis 90 Gew.-%, insbesondere im Bereich von 5 bis 80 Gew.-%, vorzugsweise im Bereich von 10 bis 70 Gew.-%, bevorzugt im Bereich von 20 bis 65 Gew.-%, besonders bevorzugt im Bereich von 30 bis 60 Gew.-%, enthält. Erfindungsgemäß wird unter der Gesamtmenge die Summe der Menge aller in der Zusammensetzung enthaltenen Pflanzenextrakte verstanden.

Gleichermaßen kann es im Rahmen der vorliegenden Erfindung vorgesehen sein, dass die Zusammensetzung Pflanzenextrakt(e) bzw. die Komponente (b) in einer absoluten Gesamtmenge im Bereich von 0,1 bis 1.000 ml, insbesondere im Bereich von 1 bis 500 ml, vorzugsweise im Bereich von 5 bis 250 ml, bevorzugt im Bereich von 10 bis 100 ml, besonders bevorzugt im Bereich von 20 bis 80 ml, noch mehr bevorzugt im Bereich von 30 bis 60 ml, enthält, bezogen auf eine Applikationseinheit der Zusammensetzung. Wie oben bereits definiert, wird auch in diesem Zusammenhang unter einer Applikationseinheit die Menge an Zusammensetzung verstanden, welche im Rahmen einer Einzeldosis bzw. Einzelapplikation an den Patienten verabreicht wird.

Was darüber hinaus die jeweiligen einzelnen eingesetzten Pflanzenextrakte anbelangt, so sind diese insbesondere in den nachfolgend definierten Mengen in der erfindungsgemäßen Zusammensetzung enthalten:
Was den Kamillenblüten-Extrakt anbelangt, so ist es erfindungsgemäß vorgesehen, dass die Zusammensetzung Kamillenblüten-Extrakt bzw. die Komponente (b1) in einer relativen Menge, bezogen auf die Zusammensetzung, im Bereich von 10 bis 40 Gew.-%, besonders bevorzugt im Bereich von 15 bis 30 Gew.-%, enthält.

Darüber hinaus kann es vorgesehen sein, dass die Zusammensetzung Kamillenblüten-Extrakt bzw. die Komponente (b1) in einer absoluten Menge, bezogen auf eine Applikationseinheit der Zusammensetzung, im Bereich von 0,1 bis 70 ml, insbesondere im Bereich von 1 bis 60 ml, vorzugsweise im Bereich von 5 bis 50 ml, bevorzugt im Bereich von 10 bis 40 ml, besonders bevorzugt im Bereich von 15 bis 30 ml, enthält.

Weiterhin enthält die Zusammensetzung nach der vorliegenden Erfindung Kümmelfrüchte-Extrakt bzw. die Komponente (b2) in einer relativen Menge, bezogen auf die Zusammensetzung, im Bereich von 2 bis 20 Gew.-%, besonders bevorzugt im Bereich von 5 bis 15 Gew.-%.

Gleichermaßen kann die Zusammensetzung Kümmelfrüchte-Extrakt bzw. die Komponente (b2) in einer absoluten Menge, bezogen auf eine Applikationseinheit der Zusammensetzung, im Bereich von 0,1 bis 50 ml, insbesondere im Bereich von 0,5 bis 40 ml, vorzugsweise im Bereich von 1 bis 30 ml, bevorzugt im Bereich von 2 bis 20 ml, besonders bevorzugt im Bereich von 5 bis 15 ml, enthalten.

Zudem ist es im Rahmen der vorliegenden Erfindung vorgesehen, dass die Zusammensetzung Melissenblätter-Extrakt bzw. die Komponente (b3) in einer relativen Menge, bezogen auf die Zusammensetzung, im Bereich von 2 bis 20 Gew.-%, besonders bevorzugt im Bereich von 5 bis 15 Gew.-%, enthält.

Gleichermaßen kann es vorgesehen sein, dass die Zusammensetzung Melissenblätter-Extrakt bzw. die Komponente (b3) in einer absoluten Menge, bezogen auf eine Applikationseinheit der Zusammensetzung, im Bereich von 0,1 bis 50 ml, insbesondere im Bereich von 0,5 bis 40 ml, vorzugsweise im Bereich von 1 bis 30 ml, bevorzugt im Bereich von 2 bis 20 ml, besonders bevorzugt im Bereich von 5 bis 15 ml, enthält.

Was schließlich den Pfefferminzblätter-Extrakt bzw. die Komponente (b4) anbelangt, so enthält die Zusammensetzung diesen bzw. diese in einer relativen Menge, bezogen auf die Zusammensetzung, im Bereich von 1 bis 10 Gew.-%, besonders bevorzugt im Bereich von 2,5 bis 7,5 Gew.-%, enthalten.

Darüber hinaus kann es vorgesehen sein, dass die Zusammensetzung Pfefferminzblätter-Extrakt bzw. die Komponente (b4) in einer absoluten Menge, bezogen auf eine Applikationseinheit der Zusammensetzung, im Bereich von 0,1 bis 40 ml, insbesondere im Bereich von 0,5 bis 30 ml, vorzugsweise im Bereich von 0,7 bis 20 ml, bevorzugt im Bereich von 1 bis 10 ml, besonders bevorzugt im Bereich von 2,5 bis 7,5 ml, enthält.

In Bezug auf den Angelikawurzel-Extrakt hat es sich als wirkungsvoll erwiesen, wenn die Zusammensetzung Angelikawurzel-Extrakt bzw. die Komponente (b5) in einer relativen Menge, bezogen auf die Zusammensetzung, im Bereich von 0,1 bis 50 Gew.-%, insbesondere im Bereich von 0,5 bis 40 Gew.-%, vorzugsweise im Bereich von 1 bis 30 Gew.-%, bevorzugt im Bereich von 2 bis 20 Gew.-%, besonders bevorzugt im Bereich von 5 bis 15 Gew.-%, enthält.

Gleichermaßen kann es vorgesehen sein, dass die Zusammensetzung Angelikawurzel-Extrakt bzw. die Komponente (b5) in einer absoluten Menge, bezogen auf eine Applikationseinheit der Zusammensetzung, im Bereich von 0,1 bis 50 ml, insbesondere im Bereich von 0,5 bis 40 ml, vorzugsweise im Bereich von 1 bis 30 ml, bevorzugt im Bereich von 2 bis 20 ml, besonders bevorzugt im Bereich von 5 bis 15 ml, enthält.

Gegenstand der vorliegenden Erfindung ist somit eine pharmazeutische Zusammensetzung, welche vorzugsweise zur prophylaktischen bzw. kurativen Behandlung von Erkrankungen des Magen- und/oder Darmtrakts bzw. von Gastrointestinalerkrankungen geeignet ist, insbesondere eine wie zuvor beschriebene Zusammensetzung, welche - in Kombination und in jeweils wirksamen, insbesondere pharmazeutisch wirksamen Mengen -
(a) Hyaluronsäure oder deren Salze ("Komponente (a)"); und
(b) eine Kombination der folgenden Pflanzenextrakte:
   (b1) Kamillenblüten-Extrakt in einer relativen Menge, bezogen auf die Zusammensetzung, im Bereich von 10 bis 40 Gew.-%, besonders bevorzugt im Bereich von 15 bis 30 Gew.-%;
   (b2) Kümmelfrüchte-Extrakt in einer relativen Menge, bezogen auf die Zusammensetzung, im Bereich von 2 bis 20 Gew.-%, besonders bevorzugt im Bereich von 5 bis 15 Gew.-%;
   (b3) Melissenblätter-Extrakt in einer relativen Menge, bezogen auf die Zusammensetzung, im Bereich von 2 bis 20 Gew.-%, besonders bevorzugt im Bereich von 5 bis 15 Gew.-%;
   (b4) Pfefferminzblätter-Extrakt in einer relativen Menge, bezogen auf die Zusammensetzung, im Bereich von 1 bis 10 Gew.-%, besonders bevorzugt im Bereich von 2,5 bis 7,5 Gew.-%;
   gegebenenfalls (b5) Angelikawurzel-Extrakt, insbesondere in einer relativen Menge, bezogen auf die Zusammensetzung, im Bereich von 0,1 bis 50 Gew.-%, insbesondere im Bereich von 0,5 bis 40 Gew.-%, vorzugsweise im Bereich von 1 bis 30 Gew.-%, bevorzugt im Bereich von 2 bis 20 Gew.-%, besonders bevorzugt im Bereich von 5 bis 15 Gew.-%;
enthält.

Mit einer Zusammensetzung auf Basis der oben genannten Komponenten ist eine hervorragende Linderung von Beschwerden des Magen/Darm-Trakts, wie Gastritis, /Sodbrennen, Völlegefühl, Magendruck etc., möglich. Darüber hinaus wird die bereits geschädigte Magenschleimhaut bzw. Magenwand durch den Aufbau einer Muzinschicht vor dem stark sauren Magensaft geschützt und bei der Regeneration unterstützt. Weiterhin werden in äußerst effizienter Weise die mit Gastrointestinalerkrankungen einhergehenden Schmerzen, Entzündungen sowie Krämpfe gelindert.

Neben den eingesetzten Mengen als solchen spielt darüber hinaus auch das eingesetzte gewichtsbezogene Mengenverhältnis von saurem Glykosaminoglykan, insbesondere der Hyaluronsäure bzw. deren Salze, zu den eingesetzten Pflanzenextrakten für die Wirksamkeit der erfindungsgemäßen Zusammensetzungen eine zentrale Rolle:
In diesem Zusammenhang hat es sich als besonders vorteilhaft erwiesen, wenn die Zusammensetzung die Komponente (a) und den mindestens einen Pflanzenextrakt (b), insbesondere die Komponenten (b1) bzw. (b2) bzw. (b3) bzw. (b4) bzw. gegebenenfalls (b5) in ihrer Gesamtheit, in einem gewichtsbezogenen Verhältnis von Komponente (a) zu Komponente (b), insbesondere in einem gewichtsbezogenen Verhältnis von Komponente (a) zu der Summe der Komponenten (b1) und/oder (b2) und/oder (b3) und/oder (b4) und/oder gegebenenfalls (b5), im Bereich von 10 : 1 bis 1 : 1.000, insbesondere im Bereich von 2 : 1 bis 1 : 500, vorzugsweise im Bereich von 1 : 1 bis 1 : 100, bevorzugt im Bereich von 1 : 5 bis 1 : 75, besonders bevorzugt im Bereich von 1 : 10 bis 1 : 50, enthält.

Darüber hinaus lässt sich die Wirksamkeit der erfindungsgemäßen Zusammensetzung gezielt steuern bzw. steigern, wenn die eingesetzten Pflanzenextrakte ein definiertes bzw. zielgerichtet eingestelltes Droge/Extrakt-Verhältnis aufweisen:
Das sogenannte Droge/Extrakt-Verhältnis (DEV) charakterisiert im Rahmen der vorliegenden Erfindung das (gewichtsbezogene) Verhältnis von eingesetzter Ausgangsdroge zu erhaltenem Extrakt. Das Droge/Extrakt-Verhältnis (DEV) gibt also an, aus welcher gewichtsbezogenen Menge an eingesetzter Droge (zum Beispiel Kamillenblütenextrakt) welche gewichtsbezogene Menge an Extrakt gewonnen ist. Ein Droge/Extrakt-Verhältnis beispielsweise von 10 : 1 bedeutet, dass aus 10 Gewichtsteilen Droge 1 Gewichtsteil Extrakt gewonnen wurde. Das Droge/Extrakt-Verhältnis gibt also an, wie viele Gewichtsteile einer Arzneidroge für die Herstellung des gewichtsbezogenen Extrakt-Äquivalents benötigt werden.

Die Verwendung von Extrakten mit definiertem Drogen/Extrakt-Verhältnis ist insbesondere vor dem Hintergrund relevant, dass die Qualität bzw. Stärke des Extraktes einen entscheidenden Einfluss auf die Gesamtqualität bzw. Wirksamkeit der pharmazeutischen Zusammensetzung hat. Ziel ist dabei zudem, den Extrakt hinsichtlich der Wirkstoffkonzentration zu standardisieren, so dass auch die finale pharmazeutische Zusammensetzung qualitativ und quantitativ mit konstant guten Werten bezüglich der Wirk- und Inhaltsstoffe bereitgestellt werden kann.

In Bezug auf die Wirksamkeit der erfindungsgemäß eingesetzten Pflanzenextrakte in der Zusammensetzung hat es sich insgesamt als vorteilhaft erwiesen, wenn der mindestens eine Pflanzenextrakt, insbesondere der Angelikawurzel-Extrakt, Kamillenblüten-Extrakt, Kümmelfrüchte-Extrakt, Melissenblätter-Extrakt und/oder Pfefferminzblätter-Extrakt, jeweils ein Droge/Extrakt-Verhältnis im Bereich von 10:1 bis 1 : 100, insbesondere im Bereich von 5 : 1 bis 1 : 50, vorzugsweise im Bereich von 2 : 1 bis 1 : 10, bevorzugt im Bereich von 1 : 1 bis 1 : 5, aufweist.

In diesem Zusammenhang ist es gleichermaßen bevorzugt, wenn der mindestens eine Pflanzenextrakt, insbesondere der Angelikawurzel-Extrakt, Kamillenblüten-Extrakt, Kümmelfrüchte-Extrakt, Melissenblätter-Extrakt und/oder Pfefferminzblätter-Extrakt, jeweils ein Droge/Extrakt-Verhältnis von mindestens 1 : 50, insbesondere mindestens 1 : 10, vorzugsweise mindestens 1 : 5, bevorzugt mindestens 1 : 4, aufweist.

Gemäß einer besonderen Ausführungsform kann es vorgesehen sein, dass die Zusammensetzung nach der vorliegenden Erfindung neben den vorgenannten Inhahaltsstoffen auf Basis der Komponenten (a) und (b) zusätzlich Römische Kamille, insbesondere in Form einer homöopathischen Urtinktur, enthält. Die Römische Kamille (Chamaemelum nobile) ist eine Pfalnzenart der Korbblütler (Asteraceae) und wird wie die Echte Kamille als Heilpflanze verwendet. Die Römische Kamille enthält als wirksame Wirk- bzw. Inhaltsstoffe insbesondere Sesquiterpenlactone, insbesondere Nobilin, daneben Flavonoide, Pinen, Limonen und Bisabolol.

Gemäß einer besonders bevorzugten Ausführungsform der vorliegenden Erfindung kann es zudem vorgesehen sein, dass die Zusammensetzung einen Alkoholgehalt, insbesondere einen Ethanolgehalt, von weniger als 5 Vol.-%, insbesondere weniger als 3,0 Vol.-%, vorzugsweise weniger als 2,0 Vol.-%, bevorzugt weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt von etwa 0 Vol.-%, aufweist. Es wird erfindungsgemäß also bevorzugt, wenn die Zusammensetzung zumindest im Wesentlichen alkoholfrei, insbesondere zumindest im Wesentlichen ethanolfrei, ausgebildet ist.

Der Begriff "zumindest im Wesentlichen alkoholfrei" ist diesbezüglich dahingehend zu verstehen, dass gemäß der deutschen Arzneimittelwarenverordnung der Ethanolgehalt der pharmazeutischen Zusammensetzung weniger als 0,05 g pro maximaler Einzeldosis beträgt. Weiterhin berücksichtigt der Begriff "zumindest im Wesentlichen alkoholfrei" auch die gemäß dem Europäischen Arzneibuch vorgegebene maximale Konzentration von verbleibenden Lösungsmitteln der Klasse 3, welche Ethanol umfassen, wonach eine maximale Konzentration des Ethanols von 5 g/kg bzw. 5.000 ppm im finalen Produkt gestattet ist, ohne dass die pharmazeutische Zusammensetzung als alkoholhaltig deklariert werden muss. Die Einnahme von Arzneimitteln mit Alkohol- bzw. Ethanolgehalten dieser Größenordnung ist auch bei Vorliegen der genannten Kontraindikationen unbedenklich.

Alkoholfreie, insbesondere ethanolfreie Zusammensetzungen sind insbesondere dahingehend von Vorteil, dass ihre Verträglichkeit insgesamt verbessert ist und die Gefahr einer Magen(schleimhaut-)reizung durch den Alkohol, insbesondere durch Ethanol, signifikant verringert wird. Darüber hinaus eignen sich alkoholfreie, insbesondere ethanolfreie Zusammensetzungen auch für Kinder, Schwangere, stillende Mütter sowie an Alkoholkrankheit leidende Personen.

Wenn auch die Bereitstellung der erfindungsgemäßen Zusammensetzungen in alkoholfreier, insbesondere ethanolfreier Form bevorzugt wird, ist es grundsätzlich auch möglich, dass die erfindungsgemäßen Zusammensetzungen Alkohol bzw. Ethanol enthalten.

Die Bereitstellung alkoholfreier, insbesondere ethanolfreier Zusammensetzungen erfolgt in diesem Zusammenhang vorzugsweise wie nachfolgend beschrieben:
In Bezug auf die Bereitstellung alkoholfreier bzw. ethanolfreier Zusammensetzungen kann es vorgesehen sein, dass der Angelikawurzel-Extrakt, Kamillenblüten-Extrakt, Kümmelfrüchte-Extrakt, Melissenblätter-Extrakt und/oder Pfefferminzblätter-Extrakt, unter (i) Bereitstellung eines alkoholischen, insbesondere ethanolischen Extrakts, (ii) nachfolgendem Verdampfen des alkoholischen Lösungsmittels, insbesondere des Ethanols, und (iii) abschließendem Aufnehmen des resultierenden Drogenauszugs, insbesondere des Spissum-Extrakts, in einem geeigneten, insbesondere organischen Lösungsmittel, vorzugsweise in einem glykolhaltigen Lösungsmittel, bevorzugt in Propylenglykol, erhältlich ist.

Es resultieren auf diese Weise alkoholfreie, insbesondere ethanolfreie Extrakte, welche wiederum zur Bereitstellung der erfindungsgemäßen Zusammensetzung verwendet werden können. Vorzugsweise wird als Lösungsmittel Propylenglykol eingesetzt, da sich dieses einerseits aufgrund der geringen Toxizität durch eine hervorragende Verträglichkeit auszeichnet und es andererseits neben dem sauren Glykosaminoglykan, insbesondere der Hyaluronsäure, eine weitere Konsistenzsteuerung der erfindungsgemäßen Zusammensetzung ermöglicht.

Weiterhin hat es sich, insbesondere im Hinblick auf die Verträglichkeit der erfindungsgemäßen Zusammensetzung, als vorteilhaft erwiesen, wenn die Zusammensetzung zumindest im Wesentlichen frei ist von (magensäure-)neutralisierenden anorganischen Komponenten, insbesondere Carbonaten, Hydrogencarbonaten und Hydroxiden, insbesondere Aluminiumhydroxid. Mit anderen Worten ist es erfindungsgemäß bevorzugt, wenn die Zusammensetzung zumindest im Wesentlichen frei ist von Antazida.

Antazida, insbesondere aluminiumhaltige Antazida, führen nämlich häufig zu unerwünschten Nebenwirkungen und Kreuzreaktionen mit anderen Pharmazeutika bzw. Arzneimitteln. Insbesondere treten nach der Einnahme aluminiumhaltiger Antazida, wie Aluminiumhydroxid, häufig unangenehme Obstipationen auf. Darüber hinaus kann es bei einer Einnahme in größeren Mengen zu Aluminium-Intoxikationen, wie z. B. Dialyse-Enzephalopathie, kommen. Aluminiumhaltige Zusammensetzungen zur Verwendung als Antazida werden beispielsweise in der EP 2 435 021 B1 beschrieben.

Um darüber hinaus zu gewährleisten, dass die erfindungsgemäße Zusammensetzung einen definierten bzw. konstanten pH-Wert aufweist, kann es erfindungsgemäß vorgesehen sein, dass die Zusammensetzung mindestens ein pH-Puffersystem aufweist. Dabei ist das Puffersystem vorzugsweise ausgewählt aus physiologisch verträglichen Puffersystemen, insbesondere Citronensäure/CitratPuffer oder Essigsäure/Acetat-Puffer, vorzugsweise Citronensäure/Citrat-Puffer.

Im Rahmen der vorliegenden Erfindung hat sich nämlich überraschend gezeigt, dass durch den Einsatz eines wie zuvor definierten pH-Puffersystems die Stabilität der Zusammensetzung, insbesondere die Langzeitstabilität, signifikant verbessert werden kann, wie es auch durch die nachfolgend geschilderten Ausführungsbeispiele belegt wird. Insbesondere bei Einsatz von Citronensäure/Citrat-Puffer kann die Stabilität der Zusammensetzungen signifikant gesteigert werden.

Neben der Auswahl des Puffersystems als solchem spielt zudem der eingestellte pH-Wert der erfindungsgemäßen Zusammensetzung eine zentrale Rolle in Bezug auf die Stabilität der Komponenten. In diesem Zusammenhang ist es insbesondere bevorzugt, wenn die Zusammensetzung einen physiologisch verträglichen pH-Wert aufweist, insbesondere einen pH-Wert im Bereich von 4 bis 9, insbesondere im Bereich von 5 bis 8, vorzugsweise im Bereich von 5,5 bis 6.

Um darüber hinaus das Risiko eines mikrobiologischen Befalls zu minimieren, kann es erfindungsgemäß vorgesehen sein, dass die Zusammensetzung mindestens ein pharmazeutisch verträgliches Konservierungsmittel enthält. Vorzugsweise ist das Konservierungsmittel ausgewählt aus Phenolen und deren Derivaten, insbesondere Benzylalkoholen, Chlorbutanol bzw. Propylenglykol, aliphatischen und aromatischen Alkoholen, insbesondere Kresolen, Parabenen und/oder Chlorkresolen, quartären Ammoniumverbindungen, insbesondere Benzalkoniumchlorid und/oder Cetylpyridiniumchlorid, und Carbonsäuren und deren Salzen, insbesondere Sorbinsäure und/oder Benzoesäure und/oder deren Salzen. Ganz besonders bevorzugt als Konservierungsmittel ist Sorbinsäure und deren Salze (Sorbate), insbesondere Alkalisorbat (z. B. Kaliumsorbat); hiermit werden die lagerstabilsten Zusammensetzungen erhalten.

Was die Mengen des Konservierungsmittels anbelangt, so enthält die Zusammensetzung dieses vorzugsweise in einer relativen Menge im Bereich von 0,001 bis 3 Gew.-%, insbesondere 0,01 bis 2 Gew.-%, vorzugsweise im Bereich von 0,05 bis 1 Gew.-%,bevorzugt im Bereich von 0,1 bis 0,5 Gew.-%, bezogen auf die Zusammensetzung.

Gemäß einer besonders bevorzugten Ausführungsform der vorliegenden Erfindung ist es bevorzugt, wenn das Konservierungsmittel Sorbinsäure bzw. ein Salz der Sorbinsäure ist, insbesondere das Natrium- oder Kaliumsalz der Sorbinsäure. Durch den Einsatz von Sorbinsäure bzw. dessen Salzen, insbesondere Kalium- bzw. Natriumsorbat, kann eine hervorragende Wirkung gegenüber pathogenen Keimen bzw. Bakterien erzielt werden, was wiederum die Stabilität der Zusammensetzung verbessert. Darüber hinaus hat sich in diesem Zusammenhang gezeigt, dass Kaliumsorbat, insbesondere in den erfindungsgemäß bevorzugten pH-Bereichen, eine verbesserte Stabilität in Citronensäure/Citrat-Puffer besitzt. Durch den Einsatz von Kaliumsorbat in Kombination mit dem erfindungsgemäß ausgewählten pH-Bereich und Puffersystem kann somit die Stabilität, insbesondere die Langzeitstabilität, der erfindungsgemäßen Zusammensetzung weiterführend gesteigert werden.

Darüber hinaus ist es erfindungsgemäß bevorzugt, wenn die Zusammensetzung Wasser, insbesondere gereinigtes Wasser, in einer Menge im Bereich von 1 bis 70 Gew.-%, insbesondere im Bereich von 5 bis 50 Gew.-%, bevorzugt im Bereich von 10 bis 40 Gew.-%, besonders bevorzugt im Bereich von 15 bis 30 Gew.-%, bezogen auf die Zusammensetzung, enthält. Insbesondere kann es vorgesehen sein, dass die Zusammensetzung Wasser als pharmazeutisch verträglichen Träger (Exzipienten) enthält. In diesem Zusammenhang kann es somit insbesondere vorgesehen sein, dass die Zusammensetzung wässrig basiert ausgebildet ist.

Was die physikalische Ausgestaltung der erfindungsgemäßen Zusammensetzung anbelangt, so kann diese in den üblichen Formen vorzugsweise pharmazeutischer Zusammensetzungen vorliegen. Erfindungsgemäß bevorzugt ist es, wenn die Zusammensetzung flüssig oder viskos, insbesondere viskos, vorzugsweise gelförmig, ausgebildet ist. Insbesondere kann es vorgesehen sein, dass die Zusammensetzung in Form eines Gels vorliegt.

Unter dem Begriff "Gel" bzw. "gelförmig" wird im Rahmen der vorliegenden Erfindung ein feindisperses System aus mindestens einer festen und einer flüssigen Phase verstanden, wobei die feste Phase dabei ein schwammartiges, dreidimensionales Netzwerk bildet, dessen Poren durch eine flüssige Phase ausgefüllt werden, so dass insgesamt eine zähflüssige bzw. viskose Masse ausgebildet wird. Im Rahmen der vorliegenden Erfindung kann die Gelbildung insbesondere durch die eingesetzte Hyaluronsäure erfolgen. Die gelförmige bzw. viskose Ausbildung der erfindungsgemäßen Zusammensetzung ist mit dem Vorteil verbunden, dass eine besonders effiziente und langanhaltende Auskleidung der Magenschleimhaut erzielt wird.

Neben der Gelbildung durch das saure Glykosaminoglykan kann es auch vorgesehen sein, dass die Zusammensetzung zusätzlich mindestens einen Gelbildner aufweist. Gemäß einer erfindungsgemäßen Ausführungsform kann es in diesem Zusammenhang vorgesehen sein, dass der Gelbildner aus der Gruppe von Bentoniten, Kieselsäuren, Polyacrylsäuren, Carbomeren, Polyvinylpyrrolidonen, Cellulose und Cellulosederivaten, insbesondere Estern und Ethern der Cellulose, Xanthanen sowie deren Mischungen ausgewählt ist.

Was die eingesetzten Mengen an Gelbildner anbelangt, so können diese im Rahmen der vorliegenden Erfindung in weiten Bereichen variieren. Besonders gute Ergebnisse werden jedoch erzielt, wenn die Zusammensetzung den Gelbildner in einer Menge im Bereich von 0,01 bis 20 Gew.-%, insbesondere im Bereich von 0,1 bis 10 Gew.-%, vorzugsweise im Bereich von 0,5 bis 5 Gew.-%, bevorzugt im Bereich von 1 bis 3 Gew.-%, bezogen auf die Zusammensetzung, aufweist.

Im Rahmen der vorliegenden Erfindung ist es somit möglich, auf Basis der ausgewählten Gelbildner sowie der einsetzbaren Mengen des Gelbildners die rheologischen Eigenschaften der Zusammensetzung nach der Erfindung gezielt einzustellen und sozusagen maßzuschneidern.

Was darüber hinaus die Viskosität der erfindungsgemäßen Zusammensetzung anbelangt, so kann diese ebenfalls in weiten Bereichen variieren. Besonders gute Ergebnisse, insbesondere in Bezug auf die Auskleidung der Magenschleimhaut mit der erfindungsgemäßen Zusammensetzung, welche eng mit der Wirksamkeit der Zusammensetzung korreliert, werden erhalten, wenn die Zusammensetzung bei einer Temperatur von 20 °C eine dynamische Viskosität im Bereich von 20 bis 20.000 mPa·s, insbesondere im Bereich von 50 bis 15.000 mPa·s, vorzugsweise im Bereich von 100 bis 10.000 mPa·s, bevorzugt im Bereich von 250 bis 8.000 mPa·s, besonders bevorzugt im Bereich von 500 bis 7.000 mPa·s, noch mehr bevorzugt im Bereich von 750 bis 6.000 mPa·s, aufweist.

Was die Methoden bzw. Verfahren zur Bestimmung der Viskosität der erfindungsgemäßen Zusammensetzung anbelangt, so sind diese als solche dem Fachmann bekannt, so dass keine weiteren diesbezüglichen Informationen an dieser Stelle notwendig sind. Insbesondere können Messgeräte, wie Kapillarviskosimeter, Rotationsviskosimeter, Kugelfall-Viskosimeter oder Brookfield-Viskosimeter zur Bestimmung der Viskosität eingesetzt werden. Insbesondere beziehen sich die zuvor angegebenen Viskositätswerte auf eine Bestimmung gemäß DIN 53015 bei Raumtemperatur (20 °C) (z. B. mittels Kugelfall-Viskosimeter, z. B. RheoTec Messtechnik GmbH, Deutschland).

Darüber hinaus kann es gemäß einer bevorzugten Ausführungsform der vorliegenden Erfindung vorgesehen sein, dass die Zusammensetzung bei einer Temperatur von 20 °C eine Dichte im Bereich von 0,5 bis 2,0 g/cm³, insbesondere im Bereich von 0,7 bis 1,8 g/cm³, vorzugsweise im Bereich von 1 bis 1,7 g/cm³, bevorzugt im Bereich von 1,1 bis 1,6 g/cm³, aufweist.

Weiterhin ist es möglich, dass die Zusammensetzung mindestens einen weiteren Wirk- und/oder Inhaltsstoff, insbesondere ausgewählt aus der Gruppe von (Schleim)Hautschutzmitteln, Antiseptika, Lokalanästhetika, Vitaminen, Spurenelementen, Mineralien, Mikronährstoffen sowie deren Kombinationen, enthält.

Darüber hinaus kann es vorgesehen sein, dass die Zusammensetzung mindestens einen üblichen pharmazeutischen Zusatz- und/oder Hilfsstoff enthält. Dieser kann insbesondere aus der Gruppe von Verarbeitungshilfsstoffen, Stabilisatoren, Emulgatoren, Antioxidantien, Konservierungsstoffen, Feuchthaltemitteln, pH-Stellmitteln, pH-Puffersubstanzen, Verdickungsmitteln, Antiseptika, Färbe-, Puffer-, Riech-, Duft-, Streck-, Binde-, Netz- und/oder Konservierungsstoffen, geschmacksverbessernden Additiven, Aromen und Süßungsmitteln sowie deren Kombinationen ausgewählt sein.

Im Allgemeinen eignet sich die erfindungsgemäße Zusammensetzung zur Verwendung im Bereich der Medizin, Pharmazie und Lebensmitteltechnik.

Gegenstand der vorliegenden Erfindung ist somit insbesondere eine Zusammensetzung, wie sie zuvor beschrieben wurde, zur Verwendung bei der prophylaktischen und/oder kurativen Behandlung von Erkrankungen und/oder Beschwerden des Magen- und/oder Darmtrakts, insbesondere Magenschmerzen, Völlegefühl, Blähungen, Magen- und Darmkrämpfen, Übelkeit, Sodbrennen, Reizmagen, Gastritis, Hyperazidität, Ösophagitis, dyspeptischen Beschwerden, Magengeschwüren, Magendruck, Speiseunverträglichkeiten und/oder Schleimhautschädigungen.

Ein weiterer Gegenstand der vorliegenden Erfindung - gemäß einem **zweiten** Aspekt der vorliegenden Erfindung - ist ein Behältnis, insbesondere eine Applikationsvorrichtung, vorzugsweise in Form einer Pumpflasche oder eines Dosierbeutels, insbesondere in Form einer Pumpflasche, welche eine erfindungsgemäße Zusammensetzung, wie sie zuvor beschrieben wurde, enthält.

Grundsätzlich kann die erfindungsgemäße Zusammensetzung in jeglichen dem Fachmann für insbesondere pharmazeutische Zwecke bekannten Behältnissen bzw. Applikationsvorrichtungen vorliegen. Der Einsatz einer Pumpflasche ist jedoch mit dem Vorteil einer guten Dosierbarkeit und Lagerbarkeit verbunden. Darüber hinaus lassen sich angebrochene Behältnisse bzw. Pumpflaschen einfach wiederverschließen, so dass es nicht zu einer Verunreinigung der Zusammensetzung in dem Behältnis bzw. der Pumpflasche kommt.

Für weitergehende Einzelheiten zu diesem Erfindungsaspekt kann auf die vorstehenden Ausführungen zu dem ersten erfindungsgemäßen Aspekt verwiesen werden, welche entsprechend auch in Bezug auf diesen Erfindungsaspekt gelten.

Weiterhin betrifft die vorliegende Erfindung - gemäß einem **dritten** Aspekt der vorliegenden Erfindung - ein Arzneimittel, insbesondere zur prophylaktischen und/oder kurativen Behandlung bzw. zur Verwendung bei der prophylaktischen und/oder kurativen Behandlung von Erkrankungen und/oder Beschwerden des Magen- und/oder Darmtrakts, insbesondere Magenschmerzen, Völlegefühl, Blähungen, Magen- und Darmkrämpfen, Übelkeit, Sodbrennen, Reizmagen, Gastritis, Hyperazidität, Ösophagitis, dyspeptischen Beschwerden, Magengeschwüren, Magendruck, Speiseunverträglichkeiten und/oder Schleimhautschädigungen, welches eine erfindungsgemäße Zusammensetzung, wie sie zuvor beschrieben wurde, enthält.

Für weitergehende Einzelheiten zu diesem Erfindungsaspekt kann auf die vorstehenden Ausführungen zu dem ersten und zweiten erfindungsgemäßen Aspekt verwiesen werden, welche entsprechend auch in Bezug auf diesen Erfindungsaspekt gelten.

Die zuvor beschriebene Zusammensetzung eignet sich zur Verwendung im Bereich der Medizin, Pharmazie und Lebensmitteltechnik.

Insbesondere kann die zuvor beschriebene Zusammensetzung zur prophylaktischen und/oder kurativen Behandlung von Erkrankungen und/oder Beschwerden des Magen- und/oder Darmtrakts, insbesondere Magenschmerzen, Völlegefühl, Blähungen, Magen- und Darmkrämpfen, Übelkeit, Sodbrennen, Reizmagen, Gastritis, Hyperazidität, Ösophagitis, dyspeptischen Beschwerden, Magengeschwüren, Magendruck, Speiseunverträglichkeiten und/oder Schleimhautschädigungen, verwendet werden.

### Ausführungsbeispiele:

### 1. Bereitstellung von erfindungsgemäßen Zusammensetzungen und von Vergleichszusam mensetzungen

Um die Zusammensetzungen nach der vorliegenden Erfindung in Bezug auf ihre Wirksamkeit, ihre Wirkeffizienz, ihre Verträglichkeit sowie ihre Stabilität zu untersuchen, wurden erfindungsgemäße Zusammensetzungen sowie Vergleichszusammensetzungen bereitgestellt. Alle eingesetzten Pflanzenextrakte sind jeweils ethanolfrei ausgebildet und weisen jeweils ein Droge/Extrakt-Verhältnis von etwa 1 : 2,5-3,5 auf. Die eingesetzte Hyaluronsäure weist ein (mittleres) Molekulargewicht von ca. 1,7 MDa auf. Alle Zusammensetzungen waren mit Citronensäure/Citrat-Puffer auf einen pH-Wert von ca. 5,5 eingestellt.

### Zusammensetzung A (erfindungsgemäß):

In an sich bekannter Weise wurde eine erfindungsgemäße Zusammensetzung A in Form eines Magengels bereitgestellt:

| **Inhaltsstoffe** | **Menge in Gew.-%** |
|---|---|
| Angelikawurzel-Extrakt | 5 - 15 |
| Kamillenblüten-Extrakt | 15 - 30 |
| Kümmelfrüchte-Extrakt | 5 - 15 |
| Melissenblätter-Extrakt | 5 - 15 |
| Pfefferminzblätter-Extrakt | 2,5 - 7,5 |
| Natrium-Hyaluronat | 1 - 2 |
| Kaliumsorbat | 0,1 - 0,5 |
| sonstige Hilfsstoffe | 18 - 25 |
| gereinigtes Wasser | 15 - 30 |

Die obige Zusammensetzung wies einen pH-Wert von 5,5 auf.

### Zusammensetzung A' (erfindungsgemäß):

In an sich bekannter Weise wurde eine erfindungsgemäße Zusammensetzung A' in Form eines Magengels bereitgestellt:

| **Inhaltsstoffe** | **Menge in Gew.-%** |
|---|---|
| Kamillenblüten-Extrakt | 15 - 30 |
| Kümmelfrüchte-Extrakt | 5 - 15 |
| Melissenblätter-Extrakt | 5 - 15 |
| Pfefferminzblätter-Extrakt | 2,5 - 7,5 |
| Natrium-Hyaluronat | 1 - 2 |
| Kaliumsorbat | 0,1 - 0,5 |
| sonstige Hilfsstoffe | 1 - 20 |
| gereinigtes Wasser | 20 - 80 |

Die obige Zusammensetzung wies einen pH-Wert von 5,5 auf.

### Zusammensetzung B (erfindungsgemäß):

In an sich bekannter Weise wurde eine weitere erfindungsgemäße Zusammensetzung B in Form eines Magengels hergestellt:

| **Inhaltsstoffe** | **Menge in Gew.-%** |
|---|---|
| Angelikawurzel-Extrakt | 5 - 15 |
| Kamillenblüten-Extrakt | 15 - 30 |
| Pfefferminzblätter-Extrakt | 2,5 - 7,5 |
| Natrium-Hyaluronat | 1 - 2 |
| Kaliumsorbat | 0,1 - 0,5 |
| sonstige Hilfsstoffe | 18 - 25 |
| gereinigtes Wasser | 20 - 40 |

Die oben aufgeführte Zusammenfassung B wies einen pH-Wert von 5,5 auf.

### Zusammensetzung C (erfindungsgemäß):

In an sich bekannter Weise wurde eine erfindungsgemäße Zusammensetzung C in Form eines Magengels hergestellt:

| **Inhaltsstoffe** | **Menge in Gew.-%** |
|---|---|
| Angelikawurzel-Extrakt | 5 - 15 |
| Natrium-Hyaluronat | 1 - 2 |
| Kaliumsorbat | 0,1 - 0,5 |
| sonstige Hilfsstoffe | 18 - 25 |
| gereinigtes Wasser | 30 - 70 |

Die erfindungsgemäße Zusammensetzung C wies einen pH-Wert von 5,5 auf.

### Zusammensetzung D (Vergleich):

In an sich bekannter Weise wurde eine Vergleichszusammensetzung D in Form eines Magengels hergestellt:

| **Inhaltsstoffe** | **Menge in Gew.-%** |
|---|---|
| Natriumhyaluronat | 1 - 2 |
| Kaliumsorbat | 0,1 - 0,5 |
| sonstige Hilfsstoffe | 18 - 25 |
| gereinigtes Wasser | 60 - 80 |

Die obige Zusammensetzung wies ebenfalls einen pH-Wert von 5,5 auf und enthielt als einzigen Wirkstoff Natriumhyaluronat.

### Zusammensetzung E (Vergleich):

In an sich bekannter Weise wurde schließlich eine Vergleichszusammensetzung E in Form eines Magengels hergestellt:

| **Inhaltsstoffe** | **Menge in Gew.-%** |
|---|---|
| Angelikawurzel-Extrakt | 5 - 15 |
| Kamillenblüten-Extrakt | 15 - 30 |
| Kümmelfrüchte-Extrakt | 5 - 15 |
| Melissenblätter-Extrakt | 5 - 15 |
| Pfefferminzblätter-Extrakt | 2,5 - 7,5 |
| Kaliumsorbat | 0,1 - 0,5 |
| sonstige Hilfsstoffe | 18 - 25 |
| gereinigtes Wasser | 15 - 30 |

Die obige Zusammensetzung E wies ebenfalls einen pH-Wert von 5,5 auf und enthielt als pharmakologisch relevante Inhaltsstoffe die erfindungsgemäß bevorzugten Pflanzenextrakte, jedoch keine Hyaluronsäure.

### 2. Wirksamkeitsstudien zur Behandlung von Gastrointestinalbeschwerden

Um die Wirksamkeit und Verträglichkeit der erfindungsgemäßen Zusammensetzungen zu untersuchen, wurden im Rahmen einer Wirksamkeitsstudie insgesamt 60 Patienten im Alter von 45 bis 70 Jahren, von denen 29 weiblich und 31 männlich waren, herangezogen. Die Probanden litten bereits seit mindestens 6 Monaten regelmäßig an Magenschleimhautreizungen sowie den damit einhergehenden Beschwerden, insbesondere Magenschmerzen und Sodbrennen. Es wurden fünf Untersuchungsgruppen von jeweils 10 Personen gebildet.

Die Probanden bekamen jeweils eine der Zusammensetzungen A, A', B, C, D und E dreimal täglich, nämlich morgens und mittags sowie abends vor dem Nachtschlaf, verabreicht. Die Therapie wurde insgesamt über einen Zeitraum von zwei Wochen durchgeführt. Die Patienten wurden dazu angehalten, die Wirksamkeit der jeweils getesteten Zusammensetzung 7 Tage nach Therapiebeginn sowie zum Abschluss der Therapie in Bezug auf die Schmerzsymptomatik, das Auftreten von Übelkeit bzw. Unwohlsein sowie eine Linderung von Sodbrennen anhand einer Schulnotenskala mit Werten von "1 = sehr gut" bis 6 = "ungenügend" zu beurteilen. Die diesbezüglichen Ergebnisse können der nachfolgenden Tabelle 1 entnommen werden. Darüber hinaus erfolgte eine abschließende Beurteilung der langfristigen Wirksamkeit in Bezug auf das Auftreten von Rückfällen bzw. Rezidiven durch die Probanden vier Wochen nach Therapieende.

**Tabelle 1: Bewertung der Wirksamkeit nach dem Schulnotensystem**

| **Zusamsammensetzung** | **7 Tage** | | | **14 Tage** | | |
|---|---|---|---|---|---|---|
| | Schmerzen | Übelkeit / Unwohlsein | Sodbrennen | Schmerzen | Übelkeit / Unwohlsein | Sodbrennen |
| A | 1-2 | 1-2 | 1-2 | 1 | 1 | 1 |
| A' | 1-2 | 1-2 | 1-2 | 1 | 1 | 1 |
| B | 2 | 2 | 1-2 | 1-2 | 1 | 1 |
| C | 2 | 2 | 2 | 1-2 | 1-2 | 1-2 |
| D | 4 | 3-4 | 4 | 3-4 | 3 | 3-4 |
| E | 3-4 | 4 | 3-4 | 3 | 3-4 | 3 |

Wie Tabelle 1 zu entnehmen ist, kann mit den erfindungsgemäßen Zusammensetzungen eine hervorragende Linderung der mit Magenschleimhautreizungen verbundenen Beschwerden, wie Schmerzen, Übelkeit und Sodbrennen, erzielt werden. Bereits nach einer kurzen Behandlungsdauer von nur 7 Tagen war bei den Probanden eine deutliche Verbesserung des Befindens zu verzeichnen. Die besten Ergebnisse wurden mit den Zusammensetzung A und A' erzielt, welche neben Hyaluronsäure als Inhaltsstoff eine Kombination vier bzw. fünf erfindungsgemäß ausgewählter Pflanzenextrakte enthielt. Insbesondere zeichneten sich die erfindungsgemäßen Zusammensetzungen auch durch eine besonders lang anhaltende Wirkung aus, d. h. auch Stunden nach Einnahme der Zusammensetzung waren die Probanden zumindest im Wesentlichen frei von Beschwerden, wie Schmerzen oder Sodbrennen. Nach Abschluss der Therapie galten alle Probanden, welche die erfindungsgemäßen Zusammensetzungen erhalten hatten, als genesen bzw. austherapiert.

Mit den Vergleichszusammensetzungen D und E hingegen konnte kein zufriedenstellender Behandlungserfolg erzielt werden. Bei Probanden, welche mit Zusammensetzung D behandelt worden waren, stellte sich zwar eine kurzfristige Linderung der Beschwerden ein, allerdings traten bereits ca. zwei Stunden nach Einnahme der Zusammensetzung wieder Beschwerden auf. Auch mit Zusammensetzung E konnte keine nennenswerte Linderung der Beschwerden im Hinblick auf eine vollständige Genesung erzielt werden.

Bei keinem der Probanden waren während der Behandlungsdauer unerwünschte Nebenwirkungen aufgetreten.

Im Rahmen der abschließenden Beurteilung der Langzeitwirksamkeit der jeweils getesteten Zusammensetzungen hat sich zudem gezeigt, dass auch langfristig eine Linderung der mit Gastrointestinalerkrankungen einhergehenden Beschwerden möglich ist. Bei keinem der Probanden, welche mit den erfindungsgemäßen Zusammensetzungen A, A' und B behandelt worden waren, war es in einem Zeitraum von vier Wochen nach Abschluss der Behandlung zu Rückfällen gekommen. Von den 10 Probanden, welche Zusammensetzung C erhalten hatten, war es bei zwei Probanden zu einem leichten Rückfall gekommen. Bei den Probanden, welche die Vergleichszusammensetzungen D und E erhalten hatten, konnte kein langfristiger Behandlungserfolg erzielt werden.

Die hervorragende Langzeitwirkung ist vermutlich - ohne sich hierbei auf diese Theorie beschränken zu wollen - auf die kombinierte Wirkweise der erfindungsgemäßen Zusammensetzungen zurückzuführen, nämlich den Wiederaufbau bzw. die Reparatur der geschädigten Muzinschicht auf der Magenschleimhaut einerseits sowie die antiphlogistischen, krampflösenden und karminativen Eigenschaften andererseits.

Die vorliegende Patientenstudie belegt die hervorragende (Langzeit-)-Wirksamkeit und Überlegenheit der erfindungsgemäßen Kombination auf Basis von Hyaluronsäure einerseits sowie den speziell ausgewählten Pflanzenextrakten andererseits. Insbesondere wird anhand der obigen Ausführungen zudem deutlich, dass die höchste Wirkeffizienz mit einer Zusammensetzung, welche als Pflanzenextrakte eine Kombination von Kamillenblüten-Extrakt, Kümmelfrüchte-Extrakt, Melissenblätter-Extrakt und Pfefferminzblätter-Extrakt sowie gegebenenfalls auch noch Angelikawurzel-Extrakt aufweist, erzielt werden kann.

### 3. Stabilitätsuntersuchungen der erfindungsgemäßen Zusammensetzungen

Darüber hinaus wurden die Zusammensetzungen auch in Bezug auf ihre Stabilität, insbesondere ihre Lagerstabilität bzw. Langzeitstabilität, untersucht. In diesem Zusammenhang wurden noch weitere Zusammensetzungen bereitgestellt, welche grundsätzlich auf der Rezeptur von Zusammensetzung A bzw. A' basierten, d. h. als Wirkstoffe Hyaluronsäure sowie Kamillenblüten-Extrakt, Kümmelfrüchte-Extrakt, Melissenblätter-Extrakt und Pfefferminzblätter-Extrakt sowie gegebenenfalls Angelikawurzel-Extrakt enthielten, jedoch in Bezug auf den pH-Wert, das Puffersystem sowie das Konservierungsmittel variierten. Eine Übersicht über die bereitgestellten Zusammensetzungen lässt sich der nachstehenden Tabelle 2 entnehmen.

**Tabelle 2: Zusammensetzungen für Stabilitätsuntersuchungen**

| **Bezeichnung** | **pH-Wert** | **Puffersystem** | **Konservierungsmittel** |
|---|---|---|---|
| A1 | 5,5 | Citronensäure/Citrat | Kaliumsorbat |
| A2 | 5,5 | " | --- |
| A3 | 5,5 | " | Benzoesäure |
| A4 | 4,0 | " | --- |
| A5 | 3,0 | " | --- |
| A6 | 6,2 | " | --- |
| A7 | 5,5 | Essigsäure/Acetat | Kaliumsorbat |
| A8 | 5,5 | " | --- |
| A9 | 5,5 | " | Benzoesäure |
| A10 | 4,5 | " | --- |
| A11 | 3,7 | " | --- |
| A12 | 5,5 | Phosphatpuffer | Kaliumsorbat |
| A13 | 5,5 | " | --- |
| A14 | 5,5 | " | Benzoesäure |
| A15 | 6,0 | " | --- |
| A16 | 7,0 | " | --- |
| A17 | 7,5 | " | --- |
| A18 | 7,9 | " | --- |

Zur Untersuchung der Stabilität der in Tabelle 2 aufgeführten Zusammensetzungen A1 bis A18 wurden nach definierten Lagerzeiträumen von 3 Monaten, 9 Monaten, 12 Monaten und 24 Monaten spezielle Prüfparameter an den Zusammensetzungen gemessen. Die Lagerung erfolgte grundsätzlich bei einer definierten Temperatur von 25 °C ± 2 °C bei einer relativen Luftfeuchte der Umgebung von 60 % ± 5 %.

Als charakteristisches Maß für die Stabilität der Zusammensetzungen wurde zum einen das Aussehen der Zusammensetzung beurteilt, wobei diesbezüglich das Auftreten von Eintrübungen bzw. eine Bildung von Niederschlägen von Relevanz waren. Eintrübungen und Niederschlagsbildung sind ein Indikator für die Bildung von Abbauprodukten bzw. für ein Ausfallen von Inhaltsstoffen in der Zusammensetzung. Zum anderen wurden die Zusammensetzungen in Bezug auf mikrobiologischen Befall untersucht. Die erhaltenen Ergebnisse bezüglich der Beurteilung des Aussehens der Zusammensetzungen A1 bis A18 sind in der nachfolgenden Tabelle 3 enthalten.

**Tabelle 3: Ergebnisse der Stabilitätsuntersuchungen (Aussehen)**

| **Bezeichnung** | **3 Monate** | **9 Monate** | **12 Monate** | **24 Monate** |
|---|---|---|---|---|
| A1 | +++ | +++ | +++ | +++ |
| A2 | +++ | +++ | +++ | ++ |
| A3 | +++ | +++ | +++ | ++ |
| A4 | +++ | ++ | ++ | + |
| A5 | ++ | + | - | - |
| A6 | +++ | ++ | + | + |
| A7 | +++ | +++ | ++ | + |
| A8 | +++ | ++ | + | + |
| A9 | +++ | ++ | ++ | + |
| A10 | +++ | ++ | + | + |
| A11 | + | - | - | - |
| A12 | +++ | ++ | + | + |
| A13 | +++ | ++ | ++ | + |
| A14 | +++ | ++ | + | + |
| A15 | +++ | ++ | ++ | + |
| A16 | + | - | - | - |
| A17 | ++ | ++ | + | + |
| A18 | + | - | - | - |

| | | | | |
|---|---|---|---|---|
| "+++" = keinerlei Niederschlagsbildung (Eintrübung) "++" = minimale Niederschlagsbildung (Eintrübung) "+" = Niederschlagsbildung (Eintrübung) sichtbar, Zusammensetzungen aber noch verwendbar "-" = starke Niederschlagsbildung (Eintrübung), Zusammensetzung nicht mehr verwendbar | | | | |

Aus den vorstehenden Ausführungen geht hervor, dass eine optimale Stabilität der Zusammensetzungen erzielt werden kann, wenn Citronensäure/CitratPuffer verwendet wird und die Zusammensetzung einen pH-Wert von etwa 5,5 aufweist (vgl. Zusammensetzungen A1, A2 und A3). Die besten Ergebnisse werden mit Zusammensetzungen erzielt, welche zudem Kaliumsorbat als Konservierungsmittel aufweisen (vgl. Zusammensetzung A1). Auch mit einem pH-Wert von 4 bzw. 6,2 ließen sich jedoch noch zufriedenstellende Ergebnisse erzielen, da die Zusammensetzungen auch nach 24 Monaten noch verwendbar waren (vgl. Zusammensetzungen A4 und A6).

Darüber hinaus eignen sich grundsätzlich auch Essigsäure/Acetat-Puffer bzw. Phosphatpuffer für die Bereitstellung stabiler Zusammensetzungen (vgl. Zusammensetzungen A7 bis A11). Bessere Ergebnisse bzw. die stabilsten Zusammensetzungen werden jedoch mit Citronensäure/Citrat-Puffer erzielt.

Der nachfolgenden Tabelle 4 können schließlich die Ergebnisse in Bezug auf die Untersuchung des mikrobiologischen Befalls der Zusammensetzungen A1 bis A18 entnommen werden:

**Tabelle 4: Ergebnisse der Untersuchung des mikrobiologischen Befalls**

| **Bezeichnung** | **3 Monate** | **9 Monate** | **12 Monate** | **24 Monate** |
|---|---|---|---|---|
| A1 | - | - | - | - |
| A2 | - | - | - | + |
| A3 | - | - | - | + |
| A4 | - | - | + | + |
| A5 | - | - | + | + |
| A6 | - | - | + | + |
| A7 | - | - | - | + |
| A8 | - | - | + | + |
| A9 | - | - | + | + |
| A10 | - | - | + | + |
| A11 | - | - | + | + |
| A12 | - | - | - | + |
| A13 | - | - | + | + |
| A14 | - | - | - | + |
| A15 | - | - | + | + |
| A16 | - | - | + | + |
| A17 | - | - | + | + |
| A18 | - | - | + | + |

| | | | | |
|---|---|---|---|---|
| "-" = frei von mikrobiologischem Befall "+" = Auftreten von mikrobiellen Verunreinigungen | | | | |

Der vorstehenden Tabelle 4 kann entnommen werden, dass nur Zusammensetzungen, welche Citronensäure/Citrat-Puffer, einen pH-Wert von etwa 5,5 sowie Kaliumsorbat als Konservierungsmittel enthielten, auch nach 24 Monaten Lagerzeit noch frei von jeglichem mikrobiologischen Befall waren (vgl. Zusammensetzung A1). Wurde stattdessen Benzoesäure als Konservierungsmittel eingesetzt (vgl. Zusammensetzung A3), wurde nach einer Lagerzeit von 24 Monaten mikrobiologischer Befall nachgewiesen. Dies ist vermutlich - ohne sich hierbei auf diese Theorie beschränken zu wollen - darauf zurückzuführen, dass Kaliumsorbat bei einem pH-Wert von 5,5 sowie beim Einsatz von Citronensäure/Citrat-Puffer in Kombination mit den übrigen erfindungsgemäßen Komponenten eine größere Stabilität und somit eine länger anhaltende Wirksamkeit gegenüber Mikroorganismen aufweist.

Zusammenfassend lässt sich somit feststellen, dass sich die erfindungsgemäßen Zusammensetzungen einerseits durch ihre hervorragende Wirksamkeit bei der Behandlung von Gastrointestinalerkrankungen, wie Magenschleimhautreizungen, Sodbrennen und Magenschmerzen, auszeichnen und zu einer hervorragenden Linderung der Beschwerden führen, dabei jedoch auch einen langanhaltenden Schutz der Magenschleimhaut bzw. der Magenwand vor der Magensäure bieten. Darüber hinaus lässt sich die Stabilität der erfindungsgemäßen Zusammensetzungen überraschend auf Basis der zielgerichteten Einstellung eines definierten pH-Werts, der zielgerichteten Auswahl eines zusätzlichen Konservierungsmittels sowie des Einsatzes eines speziell ausgewählten Puffer-Systems weiterführend optimieren.

## Patentansprüche

1. Zusammensetzung, insbesondere pharmazeutische Zusammensetzung, zur prophylaktischen und/oder kurativen Behandlung von Erkrankungen des Magen- und/oder Darmtrakts und/oder von Gastrointestinalerkrankungen, wobei die Zusammensetzung - in Kombination und in jeweils wirksamen, insbesondere pharmazeutisch wirksamen Mengen -
(a) Hyaluronsäure oder deren Salze ("Komponente (a)") in einer relativen Menge im Bereich von 0,2 bis 5 Gew.-%, bezogen auf die Zusammensetzung, und
(b) eine Kombination von
Kamillenblüten-Extrakt ("Komponente (b1)")
in einer relativen Menge, bezogen auf die Zusammensetzung, im Bereich von 10 bis 40 Gew.-%,
Kümmelfrüchte-Extrakt ("Komponente (b2)")
in einer relativen Menge, bezogen auf die Zusammensetzung, im Bereich von 2 bis 20 Gew.-%,
Melissenblätter-Extrakt ("Komponente (b3)")
in einer relativen Menge, bezogen auf die Zusammensetzung, im Bereich von 2 bis 20 Gew.-%, und
Pfefferminzblätter-Extrakt ("Komponente (b4)")
in einer relativen Menge, bezogen auf die Zusammensetzung, im Bereich von 1 bis 10 Gew.-%,
enthält.

2. Zusammensetzung nach Anspruch 1, wobei die Zusammensetzung als Komponente (b) eine Kombination von Kamillenblüten-Extrakt ("Komponente (b1)"), Kümmelfrüchte-Extrakt ("Komponente (b2)"), Melissenblätter-Extrakt ("Komponente (b3)") Pfefferminzblätter-Extrakt ("Komponente (b4)") und Angelikawurzel-Extrakt ("Komponente (b5)") aufweist.

3. Zusammensetzung nach Anspruch 1 oder 2,
wobei die Zusammensetzung die Komponente (a) in einer relativen Menge im Bereich von 0,3 bis 2,5 Gew.-%, noch mehr bevorzugt 0,5 bis 1,5 Gew.-%, bezogen auf die Zusammensetzung, enthält; und/oder
wobei die Zusammensetzung die Komponente (a) in einer absoluten Menge im Bereich von 0,01 bis 25 mg, insbesondere im Bereich von 0,1 bis 15 mg, vorzugsweise im Bereich von 0,5 bis 2,5 mg, noch mehr bevorzugt im Bereich von 0,6 bis 2 mg enthält, bezogen auf eine Applikationseinheit der Zusammensetzung; und/oder
wobei die Komponente (a) das Natriumsalz der Hyaluronsäure ist; und/oder
wobei die Komponente (a), insbesondere die Hyaluronsäure oder deren physiologisch unbedenkliche Salze, ein Molekulargewicht, insbesondere ein zahlenmittleres Molekulargewicht, im Bereich von 1 bis 100.000 kDa (Kilodalton), insbesondere im Bereich von 10 bis 50.000 kDa, vorzugsweise im Bereich von 100 bis 10.000 kDa, bevorzugt im Bereich von 750 bis 5.000 kDa, besonders bevorzugt im Bereich von 900 bis 2.000 kDa, aufweist.

4. Zusammensetzung nach einem der vorangehenden Ansprüche,
wobei die Zusammensetzung Pflanzenextrakt(e) und/oder Komponente (b) in einer relativen Gesamtmenge, bezogen auf die Zusammensetzung, im Bereich von 1 bis 90 Gew.-%, insbesondere im Bereich von 5 bis 80 Gew.-%, vorzugsweise im Bereich von 10 bis 70 Gew.-%, bevorzugt im Bereich von 20 bis 65 Gew.-%, besonders bevorzugt im Bereich von 30 bis 60 Gew.-%, enthält; und/oder
wobei die Zusammensetzung Pflanzenextrakt(e) und/oder Komponente (b) in einer absoluten Gesamtmenge im Bereich von 0,1 bis 1.000 ml, insbesondere im Bereich von 1 bis 500 ml, vorzugsweise im Bereich von 5 bis 250 ml, bevorzugt im Bereich von 10 bis 100 ml, besonders bevorzugt im Bereich von 20 bis 80 ml, noch mehr bevorzugt im Bereich von 30 bis 60 ml, enthält, bezogen auf eine Applikationseinheit der Zusammensetzung.

5. Zusammensetzung nach einem der vorangehenden Ansprüche,
wobei die Zusammensetzung Kamillenblüten-Extrakt und/oder Komponente (b1) in einer relativen Menge, bezogen auf die Zusammensetzung, im Bereich von 15 bis 30 Gew.-% enthält; und/oder
wobei die Zusammensetzung Kamillenblüten-Extrakt und/oder Komponente (b1) in einer absoluten Menge, bezogen auf eine Applikationseinheit der Zusammensetzung, im Bereich von 0,1 bis 70 ml, insbesondere im Bereich von 1 bis 60 ml, vorzugsweise im Bereich von 5 bis 50 ml, bevorzugt im Bereich von 10 bis 40 ml, besonders bevorzugt im Bereich von 15 bis 30 ml, enthält; und/oder
wobei die Zusammensetzung Kümmelfrüchte-Extrakt und/oder Komponente (b2) in einer relativen Menge, bezogen auf die Zusammensetzung, im Bereich von 5 bis 15 Gew.-% enthält; und/oder
wobei die Zusammensetzung Kümmelfrüchte-Extrakt und/oder Komponente (b2) in einer absoluten Menge, bezogen auf eine Applikationseinheit der Zusammensetzung, im Bereich von 0,1 bis 50 ml, insbesondere im Bereich von 0,5 bis 40 ml, vorzugsweise im Bereich von 1 bis 30 ml, bevorzugt im Bereich von 2 bis 20 ml, besonders bevorzugt im Bereich von 5 bis 15 ml, enthält.

6. Zusammensetzung nach einem der vorangehenden Ansprüche,
wobei die Zusammensetzung Melissenblätter-Extrakt und/oder Komponente (b3) in einer relativen Menge, bezogen auf die Zusammensetzung, im Bereich von 5 bis 15 Gew.-% enthält; und/oder
wobei die Zusammensetzung Melissenblätter-Extrakt und/oder Komponente (b3) in einer absoluten Menge, bezogen auf eine Applikationseinheit der Zusammensetzung, im Bereich von 0,1 bis 50 ml, insbesondere im Bereich von 0,5 bis 40 ml, vorzugsweise im Bereich von 1 bis 30 ml, bevorzugt im Bereich von 2 bis 20 ml, besonders bevorzugt im Bereich von 5 bis 15 ml, enthält; und/oder
wobei die Zusammensetzung Pfefferminzblätter-Extrakt und/oder Komponente (b4) in einer relativen Menge, bezogen auf die Zusammensetzung, im Bereich von 2,5 bis 7,5 Gew.-% enthält; und/oder
wobei die Zusammensetzung Pfefferminzblätter-Extrakt und/oder Komponente (b4) in einer absoluten Menge, bezogen auf eine Applikationseinheit der Zusammensetzung, im Bereich von 0,1 bis 40 ml, insbesondere im Bereich von 0,5 bis 30 ml, vorzugsweise im Bereich von 0,7 bis 20 ml, bevorzugt im Bereich von 1 bis 10 ml, besonders bevorzugt im Bereich von 2,5 bis 7,5 ml, enthält; und/oder
wobei die Zusammensetzung Angelikawurzel-Extrakt und/oder Komponente (b5) in einer relativen Menge, bezogen auf die Zusammensetzung, im Bereich von 0,1 bis 50 Gew.-%, insbesondere im Bereich von 0,5 bis 40 Gew.-%, vorzugsweise im Bereich von 1 bis 30 Gew.-%, bevorzugt im Bereich von 2 bis 20 Gew.-%, besonders bevorzugt im Bereich von 5 bis 15 Gew.-%, enthält; und/oder
wobei die Zusammensetzung Angelikawurzel-Extrakt und/oder Komponente (b5) in einer absoluten Menge, bezogen auf eine Applikationseinheit der Zusammensetzung, im Bereich von 0,1 bis 50 ml, insbesondere im Bereich von 0,5 bis 40 ml, vorzugsweise im Bereich von 1 bis 30 ml, bevorzugt im Bereich von 2 bis 20 ml, besonders bevorzugt im Bereich von 5 bis 15 ml, enthält.

7. Zusammensetzung, insbesondere pharmazeutische Zusammensetzung, zur prophylaktischen und/oder kurativen Behandlung von Erkrankungen des Magen- und/oder Darmtrakts und/oder von Gastrointestinalerkrankungen, insbesondere Zusammensetzung nach einem der vorangehenden Ansprüche, wobei die Zusammensetzung - in Kombination und in jeweils wirksamen, insbesondere pharmazeutisch wirksamen Mengen -
(a) Hyaluronsäure oder deren Salze ("Komponente (a)") in einer relativen Menge im Bereich von 0,2 bis 5 Gew.-%, bezogen auf die Zusammensetzung;
(b) eine Kombination der folgenden Pflanzenextrakte:
(b1) Kamillenblüten-Extrakt, insbesondere in einer relativen Menge, bezogen auf die Zusammensetzung, im Bereich von 10 bis 40 Gew.-%, besonders bevorzugt im Bereich von 15 bis 30 Gew.-%;
(b2) Kümmelfrüchte-Extrakt, insbesondere in einer relativen Menge, bezogen auf die Zusammensetzung, im Bereich von 2 bis 20 Gew.-%, besonders bevorzugt im Bereich von 5 bis 15 Gew.-%;
(b3) Melissenblätter-Extrakt, insbesondere in einer relativen Menge, bezogen auf die Zusammensetzung, im Bereich von 2 bis 20 Gew.-%, besonders bevorzugt im Bereich von 5 bis 15 Gew.-%;
(b4) Pfefferminzblätter-Extrakt, insbesondere in einer relativen Menge, bezogen auf die Zusammensetzung, im Bereich von 1 bis 10 Gew.-%, besonders bevorzugt im Bereich von 2,5 bis 7,5 Gew.-%;
ggf. (b5) Angelikawurzel-Extrakt, insbesondere in einer relativen Menge, bezogen auf die Zusammensetzung, im Bereich von 0,1 bis 50 Gew.-%, insbesondere im Bereich von 0,5 bis 40 Gew.-%, vorzugsweise im Bereich von 1 bis 30 Gew.-%, bevorzugt im Bereich von 2 bis 20 Gew.-%, besonders bevorzugt im Bereich von 5 bis 15 Gew.-%;
enthält.

8. Zusammensetzung nach einem der vorangehenden Ansprüche,
wobei die Zusammensetzung die Komponente (a) und den mindestens einen Pflanzenextrakt (b), insbesondere die Komponenten (b1) und/oder (b2) und/oder (b3) und/oder (b4) und/oder ggf. (b5) in ihrer Gesamtheit, in einem gewichtsbezogenen Verhältnis von Komponente (a) zu Komponente (b), insbesondere in einem gewichtsbezogenen Verhältnis von Komponente (a) zu der Summe der Komponenten (b1) und/oder (b2) und/oder (b3) und/oder (b4) und/oder ggf. (b5), im Bereich von 10 : 1 bis 1 : 1.000, insbesondere im Bereich von 2 : 1 bis 1 : 500, vorzugsweise im Bereich von 1 : 1 bis 1 : 100, bevorzugt im Bereich von 1 : 5 bis 1 : 75, besonders bevorzugt im Bereich von 1 : 10 bis 1 : 50, enthält; und/oder
wobei der mindestens eine Pflanzenextrakt, insbesondere der Angelikawurzel-Extrakt, Kamillenblüten-Extrakt, Kümmelfrüchte-Extrakt, Melissenblätter-Extrakt und/oder Pfefferminzblätter-Extrakt, jeweils ein Droge/Extrakt-Verhältnis im Bereich von 10 : 1 bis 1 : 100, insbesondere im Bereich von 5 : 1 bis 1 : 50, vorzugsweise im Bereich von 2 : 1 bis 1 : 10, bevorzugt im Bereich von 1 : 1 bis 1 : 5, aufweist; und/oder
wobei der mindestens eine Pflanzenextrakt, insbesondere der Angelikawurzel-Extrakt, Kamillenblüten-Extrakt, Kümmelfrüchte-Extrakt, Melissenblätter-Extrakt und/oder Pfefferminzblätter-Extrakt, jeweils ein Droge/Extrakt-Verhältnis von mindestens 1 : 50, insbesondere mindestens 1 : 10, vorzugsweise mindestens 1 : 5, bevorzugt mindestens 1 : 4, aufweist; und/oder
wobei die Zusammensetzung einen Alkoholgehalt, insbesondere einen Ethanolgehalt, von weniger als 5 Vol.-%, insbesondere weniger als 3,0 Vol.-%, vorzugsweise weniger als 2,0 Vol.-%, bevorzugt weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt von etwa 0 Vol.-%, aufweist und/oder wobei die Zusammensetzung zumindest im Wesentlichen alkoholfrei, insbesondere zumindest im Wesentlichen ethanolfrei, ausgebildet ist.

9. Zusammensetzung nach einem der vorangehenden Ansprüche,
wobei der mindestens eine Pflanzenextrakt, insbesondere der Angelikawurzel-Extrakt, Kamillenblüten-Extrakt, Kümmelfrüchte-Extrakt, Melissenblätter-Extrakt und/oder Pfefferminzblätter-Extrakt, erhältlich ist unter (i) Bereitstellung eines alkoholischen, insbesondere ethanolischen Extrakts, (ii) nachfolgendem Verdampfen des alkoholischen Lösungsmittels, insbesondere des Ethanols, und (iii) abschließendem Aufnehmen des resultierenden Drogenauszugs, insbesondere des Spissum-Extrakts, in einem geeigneten, insbesondere organischen Lösungsmittel, vorzugsweise in einem glykolhaltigen Lösungsmittel, bevorzugt in Propylenglykol; und/oder
wobei die Zusammensetzung zumindest im Wesentlichen frei ist von (magensäure-)-neutralisierenden anorganischen Komponenten, insbesondere Carbonaten, Hydrogencarbonaten und Hydroxiden, insbesondere Aluminiumhydroxid, und/oder wobei die Zusammensetzung zumindest im Wesentlichen frei ist von Antazida; und/oder
wobei die Zusammensetzung mindestens ein pH-Puffersystem aufweist, insbesondere wobei das Puffersystem ausgewählt ist aus physiologisch verträglichen Puffersystemen, vorzugsweise Citronensäure/Citrat-Puffer oder Essigsäure/ Acetat-Puffer, bevorzugt Citronensäure/Citrat-Puffer; und/oder
wobei die Zusammensetzung einen physiologisch verträglichen pH-Wert aufweist, insbesondere einen pH-Wert im Bereich von 4 bis 9, insbesondere im Bereich von 5 bis 8, vorzugsweise im Bereich von 5,5 bis 6.

10. Zusammensetzung nach einem der vorangehenden Ansprüche,
wobei die Zusammensetzung mindestens ein pharmazeutisch verträgliches Konservierungsmittel enthält;
insbesondere wobei das Konservierungsmittel ausgewählt ist aus Phenolen und deren Derivaten, insbesondere Benzylalkoholen, Chlorbutanol und/oder Propylenglykol, aliphatischen und aromatischen Alkoholen, insbesondere Kresolen, Parabenen und/oder Chlorkresolen, quartären Ammoniumverbindungen, insbesondere Benzalkoniumchlorid und/oder Cetylpyridiniumchlorid, und Carbonsäuren und deren Salzen, insbesondere Sorbinsäure und/oder Benzoesäure und/oder deren Salzen, besonders bevorzugt Sorbinsäure und deren Salzen (Sorbaten), insbesondere Alkalisorbat, wie Kaliumsorbat; und/oder
insbesondere wobei die Zusammensetzung das mindestens eine pharmazeutisch verträgliche Konservierungsmittel in einer relativen Menge im Bereich von 0,001 bis 3 Gew.-%, insbesondere 0,01 bis 2 Gew.-%, vorzugsweise im Bereich von 0,05 bis 1 Gew.-%,bevorzugt im Bereich von 0,1 bis 0,5 Gew.-%, bezogen auf die Zusammensetzung, enthält; und/oder
insbesondere wobei das Konservierungsmittel Sorbinsäure und/oder ein Salz der Sorbinsäure ist, insbesondere das Natrium- oder Kaliumsalz der Sorbinsäure; und/oder
wobei die Zusammensetzung Wasser, insbesondere gereinigtes Wasser, in einer Menge im Bereich von 1 bis 70 Gew.-%, insbesondere im Bereich von 5 bis 50 Gew.-%, bevorzugt im Bereich von 10 bis 40 Gew.-%, besonders bevorzugt im Bereich von 15 bis 30 Gew.-%, bezogen auf die Zusammensetzung, enthält und/oder wobei die Zusammensetzung Wasser als pharmazeutisch verträglichen Träger (Exzipienten) enthält und/oder wobei die Zusammensetzung wässrig basiert ausgebildet ist; und/oder
wobei die Zusammensetzung flüssig oder viskos, insbesondere viskos, vorzugsweise gelförmig, ausgebildet ist und/oder wobei die Zusammensetzung in Form eines Gels vorliegt.

11. Zusammensetzung nach einem der vorangehenden Ansprüche,
wobei die Zusammensetzung zusätzlich mindestens einen Gelbildner aufweist,
insbesondere wobei der Gelbildner ausgewählt ist aus der Gruppe von Bentoniten, Kieselsäuren, Polyacrylsäuren, Carbomeren, Polyvinylpyrrolidonen, Cellulose und Cellulosederivaten, insbesondere Estern und Ethern der Cellulose, Xanthanen sowie deren Mischungen; und/oder
insbesondere wobei die Zusammensetzung den Gelbildner in-einer Menge im Bereich von 0,01 bis 20 Gew.-%, insbesondere im Bereich von 0,1 bis 10 Gew.-%, vorzugsweise im Bereich von 0,5 bis 5 Gew.-%, bevorzugt im Bereich von 1 bis 3 Gew.-%, bezogen auf die Zusammensetzung, aufweist; und/oder
wobei die Zusammensetzung bei einer Temperatur von 20 °C eine dynamische Viskosität im Bereich von 20 bis 20.000 mPa·s, insbesondere im Bereich von 50 bis 15.000 mPa·s, vorzugsweise im Bereich von 100 bis 10.000 mPa·s, bevorzugt im Bereich von 250 bis 8.000 mPa·s, besonders bevorzugt im Bereich von 500 bis 7.000 mPa·s, noch mehr bevorzugt im Bereich von 750 bis 6.000 mPa·s, aufweist; und/oder
wobei die Zusammensetzung bei einer Temperatur von 20 °C eine Dichte im Bereich von 0,5 bis 2,0 g/cm³, insbesondere im Bereich von 0,7 bis 1,8 g/cm³, vorzugsweise im Bereich von 1 bis 1,7 g/cm³, bevorzugt im Bereich von 1,1 bis 1,6 g/cm³, aufweist; und/oder
wobei die Zusammensetzung mindestens einen weiteren Wirk- und/oder Inhaltsstoff, insbesondere ausgewählt aus der Gruppe von (Schleim-)-Hautschutzmitteln, Antiseptika, Lokalanästhetika, Vitaminen, Spurenelementen, Mineralien, Mikronährstoffen sowie deren Kombinationen, enthält; und/oder
wobei die Zusammensetzung mindestens einen üblichen pharmazeutischen Zusatz- und/oder Hilfsstoff, insbesondere ausgewählt aus der Gruppe von Verarbeitungshilfsstoffen, Stabilisatoren, Emulgatoren, Antioxidantien, Konservierungsstoffen, Feuchthaltemitteln, pH-Stellmitteln, pH-Puffersubstanzen, Verdickungsmitteln, Antiseptika, Färbe-, Puffer-, Riech-, Duft-, Streck-, Binde-, Netz- und/oder Konservierungsstoffen, geschmacksverbessernden Additiven, Aromen und Süßungsmitteln sowie deren Kombinationen.

12. Zusammensetzung nach einem der vorangehenden Ansprüche
zur Verwendung im Bereich der Medizin, Pharmazie und Lebensmitteltechnik und/oder zur Verwendung bei der prophylaktischen und/oder kurativen Behandlung von Erkrankungen und/oder Beschwerden des Magen- und/oder Darmtrakts, insbesondere Magenschmerzen, Völlegefühl, Blähungen, Magen- und Darmkrämpfen, Übelkeit, Sodbrennen, Reizmagen, Gastritis, Hyperazidität, Ösophagitis, dyspeptischen Beschwerden, Magengeschwüren, Magendruck, Speiseunverträglichkeiten und/oder Schleimhautschädigungen.

13. Behältnis, insbesondere Applikationsvorrichtung, vorzugsweise in Form einer Pumpflasche oder eines Dosierbeutels, insbesondere in Form einer Pumpflasche, enthaltend eine Zusammensetzung nach einem der vorangehenden Ansprüche.

14. Arzneimittel, insbesondere zur prophylaktischen und/oder kurativen Behandlung und/oder zur Verwendung bei der prophylaktischen und/oder kurativen Behandlung von Erkrankungen und/oder Beschwerden des Magen- und/oder Darmtrakts, insbesondere Magenschmerzen, Völlegefühl, Blähungen, Magen- und Darmkrämpfen, Übelkeit, Sodbrennen, Reizmagen, Gastritis, Hyperazidität, Ösophagitis, dyspeptischen Beschwerden, Magengeschwüren, Magendruck, Speiseunverträglichkeiten und/oder Schleimhautschädigungen, enthaltend eine Zusammensetzung nach einem der vorangehenden Ansprüche.

## Claims

1. A composition, specifically a pharmaceutical composition, for the prophylactic and/or curative treatment of illnesses of the gastric and/or intestinal tract and/or gastrointestinal diseases, wherein the composition - in combination and in each active, particularly pharmaceutically active quantities - contains
(a) hyaluronic acid or its salts ("component (a)") in a relative volume in the range of 0.2 to 5 wt.-%, in relation to the composition, and
(b) a combination of
chamomile blossom extract ("component (b1)")
in a relative volume, relating to the composition, in the range of 10 to 40 wt.-%,
caraway seed extract ("component (b2)")
in a relative volume, relating to the composition, in the range of 2 to 20 wt.-%,
lemon balm extract ("component (b3)")
in a relative volume, relating to the composition, in the range of 2 to 20 wt.-%, and
peppermint leaf extract ("component (b4)")
in a relative volume, relating to the composition, in the range of 1 to 10 wt.-%.

2. Composition according to claim 1, wherein the composition has as component (b) a combination of chamomile blossom extract ("component (b1)"), caraway seed extract ("component (b2)"), lemon balm extract ("component (b3)"), peppermint leaf extract ("component (b4)") and angelica root extract ("component (b5)").

3. The composition according to claim 1 or 2,
wherein the composition contains the component (a) in a relative volume in the range of 0.3 to 2.5 wt.-%, more preferably 0.5 to 1.5 wt.-%, in relation to the composition; and/or
wherein the composition contains the component (a) in an absolute volume in the range of 0.01 to 25 mg, particularly in the range of 0.1 to 15 mg, preferably in the range of 0.5 to 2.5 mg, more preferably in the range of 0.6 to 2 mg, relating to an application unit of the composition; and/or
wherein the component (a) is the sodium salt of hyaluronic acid; and/or
wherein component (a), particularly the hyaluronic acid or its physiological harmless salts has a molecular weight, particularly a number average molecular weight, in the range of 1 to 100,000 kDa (kilodaltons), particularly in the range of 10 to 50,000 kDa, preferably in the range of 100 to 10,000 kDa, more preferably in the range of 750 to 5,000 kDa, particularly preferably in the range of 900 to 2,000 kDa.

4. The composition according to one of the preceding claims,
wherein the composition contains plant extract(s) and/or component (b) in a relative total volume, in relation to the composition, in the range of 1 to 90 wt.-%, particularly in the range of 5 to 80 wt.-%, preferably in the range of 10 to 70 wt.-%, more preferably in the range of 20 to 65 wt.-%, particularly preferably in the range of 30 to 60 wt.-%; and/or
wherein the composition contains plant extract(s) and/or component (b) in an absolute total volume in the range of 0.1 to 1,000 ml, particularly in the range of 1 to 500 ml, preferably in the range of 5 to 250 ml, more preferably in the range of 10 to 100 ml, particularly preferably in the range of 20 to 80 ml, even more preferably in the range of 30 to 60 ml, in relation to an application unit of the composition.

5. The composition according to one of the preceding claims,
wherein the composition contains chamomile blossom extract and/or component (b1) in a relative volume, in relation to the composition, in the range of 15 to 30 wt.-%; and/or
wherein the composition contains chamomile blossom extract and/or component (b1) in an absolute volume, relating to an application unit of the composition, in the range of 0.1 to 70 ml, particularly in the range of 1 to 60 ml, preferably in the range of 5 to 50 ml, more preferably in the range of 10 to 40 ml, particularly preferably in the range of 15 to 30 ml; and/or
wherein the composition contains caraway seed extract and/or component (b2) in a relative volume, in relation to the composition, in the range of 5 to 15 wt.-%;
and/or
wherein the composition contains caraway seed extract and/or component (b2) in an absolute volume, relating to an application unit of the composition, in the range of 0.1 to 50 ml, particularly in the range of 0.5 to 40 ml, preferably in the range of 1 to 30 ml, more preferably in the range of 2 to 20 ml, particularly preferably in the range of 5 to 15 ml.

6. The composition according to one of the preceding claims,
wherein the composition contains lemon balm extract and/or component (b3) in a relative volume, in relation to the composition, in the range of 5 to 15 wt.-%;
and/or
wherein the composition contains lemon balm extract and/or component (b3) in an absolute volume, relating to an application unit of the composition, in the range of 0.1 to 50 ml, particularly in the range of 0.5 to 40 ml, preferably in the range of 1 to 30 ml, more preferably in the range of 2 to 20 ml, particularly preferably in the range of 5 to 15 ml; and/or
wherein the composition contains peppermint leaf extract and/or component (b4) in a relative volume, in relation to the composition, in the range of 2.5 to 7.5 wt.-%;
and/or
wherein the composition contains peppermint leaf extract and/or component (b4) in an absolute volume, relating to an application unit of the composition, in the range of 0.1 to 40 ml, particularly in the range of 0.5 to 30 ml, preferably in the range of 0.7 to 20 ml, more preferably in the range of 1 to 10 ml, particularly preferably in the range of 2.5 to 7.5 ml; and/or
wherein the composition contains angelica root extract and/or component (b5) in a relative volume, in relation to the composition, in the range of 0.1 to 50 wt.-%, particularly in the range of 0.5 to 40 wt.-%, preferably in the range of 1 to 30 wt.-%, more preferably in the range of 2 to 20 wt.-%, particularly preferably in the range of 5 to 15 wt.-%; and/or
wherein the composition contains angelica root extract and/or component (b5) in an absolute volume, relating to an application unit of the composition, in the range of 0.1 to 50 ml, particularly in the range of 0.5 to 40 ml, preferably in the range of 1 to 30 ml, more preferably in the range of 2 to 20 ml, particularly preferably in the range of 5 to 15 ml.

7. A composition, specifically a pharmaceutical composition, for the prophylactic and/or curative treatment of illnesses of the gastric and/or intestinal tract and/or gastrointestinal diseases, particularly a composition according to one of the preceding claims, wherein the composition - in combination and in each active, particularly pharmaceutically active quantities - contains
(a) hyaluronic acid or its salts ("component (a)") in a relative volume in the range of 0.2 to 5 wt.-%, in relation to the composition;
(b) a combination of the following plant extracts:
(b1) Chamomile blossom extract, particularly in a relative volume, in relation to the composition, in the range of 10 to 40 wt.-%, particularly preferably in the range of 15 to 30 wt.-%;
(b2) Caraway seed extract, particularly in a relative volume, in relation to the composition, in the range of 2 to 20 wt.-%, particularly preferably in the range of 5 to 15 wt.-%;
(b3) Lemon balm extract, particularly in a relative volume, in relation to the composition, in the range of 2 to 20 wt.-%, particularly preferably in the range of 5 to 15 wt.-%;
(b4) Peppermint leaf extract, particularly in a relative volume, in relation to the composition, in the range of 1 to 10 wt.-%, particularly preferably in the range of 2.5 to 7.5 wt.-%;
optionally
(b5) angelica root extract, particularly in a relative volume, in relation to the composition, in the range of 0.1 to 50 wt.-%, particularly in the range of 0.5 to 40 wt.-%, preferably in the range of 1 to 30 wt.-%, more preferably in the range of 2 to 20 wt.-%, particularly preferably in the range of 5 to 15 wt.-%.

8. The composition according to one of the preceding claims,
wherein the composition contains component (a) and the at least one plant extract (b), particularly the components (b1) and/or (b2) and/or (b3) and/or (b4) and/or if optionally (b5) as a whole, in a weight-related ratio of component (a) to component (b), particularly in a weight-related ratio of component (a) to the sum of components (b1) and/or (b2) and/or (b3) and/or (b4) and/or if optionally (b5), in the range of 10 : 1 to 1 : 1,000, particularly in the range of 2 : 1 to 1 : 500, preferably in the range of 1 : 1 to 1 : 100, more preferably in the range of 1 : 5 to 1 : 75, particularly preferably in the range of 1 : 10 to 1 : 50; and/or
wherein the at least one plant extract, particularly the angelica root extract,
chamomile blossom extract, caraway seed extract, lemon balm extract and/or peppermint leaf extract, each has a drug/extract ratio in the range of 10 : 1 to 1 : 100, particularly in the range of 5 : 1 to 1 : 50, preferably in the range of 2 : 1 to 1 : 10, more preferably in the range of 1 : 1 to 1 : 5; and/or
wherein the at least one plant extract, particularly the angelica root extract, chamomile blossom extract, caraway seed extract, lemon balm extract and/or peppermint leaf extract, each has a drug/extract ratio of at least 1 : 50, particularly at least 1 : 10, preferably at least 1 : 5, more preferably at least 1 : 4; and/or
wherein the composition has an alcohol content, particularly an ethanol content, of less than 5 vol%, particularly less than 3.0 vol%, preferably less than 2.0 vol%, more preferably less than 0.85 vol%, particularly preferably less than 0.5 vol%, very particularly preferably less than 0.25 vol%, even more preferably less than 0.1 vol%, most preferably of about 0 vol% and/or wherein the composition is formed at least substantially alcohol-free, particularly at least substantially ethanol-free.

9. The composition according to one of the preceding claims,
wherein the at least one plant extract, particularly the angelica root extract, chamomile blossom extract, caraway seed extract, lemon balm extract and/or peppermint leaf extract, is obtainable under (i) provision of an alcoholic, particularly ethanolic extract, (ii) subsequent evaporation of the alcoholic solvent particularly the ethanol, and (iii) then incorporating the resulting drug extract, particularly the spissum extract, into a suitable, particularly organic solvent, preferably into a glycol-containing solvent, more preferably into propylene glycol; and/or
wherein the composition is at least substantially free of (gastric acid) neutralising, inorganic components, particularly carbonates, hydrocarbonates and hydroxides, particularly aluminium hydroxide, and/or wherein the composition is at least substantially free of antacids; and/or
wherein the composition has at least a pH buffer system, particularly wherein the buffer system is chosen from physiologically compatible buffer systems, preferably a citric acid/citrate buffer or acetic acid/acetate buffer, more preferably a citric acid/citrate buffer; and/or
wherein the composition has a physiologically compatible pH value, particularly a pH value in the range of 4 to 9, particularly in the range of 5 to 8, preferably in the range of 5.5 to 6.

10. The composition according to one of the preceding claims,
wherein the composition contains at least one pharmaceutically compatible preservative;
particularly wherein the preservative is chosen from phenols and their derivatives, particularly benzyl alcohols, chlorobutanol and/or propylene glycol, aliphatic and aromatic alcohols, particularly cresols, parabens and/or chlorocresol, quaternary ammonium compounds, particularly benzalkonium chloride and/or cetylpyridinium chloride, and carbonic acids and their salts, particularly sorbic acid and/or benzoic acid and/or their salts, particularly preferably sorbic acid and their salts (sorbates), particularly alkali sorbates, such as potassium sorbate; and/or
particularly wherein the composition contains the at least one pharmaceutically compatible preservative in a relative volume in the range of 0.001 to 3 wt.-%, particularly 0.01 to 2 wt.-%, preferably in the range of 0.05 to 1 wt.-%, more preferably in the range of 0.1 to 0.5 wt.-%, in relation to the composition; and/or particularly wherein the preservative is sorbic acid and/or a salt of sorbic acid, particularly sodium or potassium salt of the sorbic acid; and/or
wherein the composition contains water, particularly purified water, in a volume in the range of 1 to 70 wt.-%, particularly in the range of 5 to 50 wt.-%, more preferably in the range of 10 to 40 wt.-%, particularly preferably in the range of 15 to 30 wt.-%, in relation to the composition, and/or wherein the composition contains water as a pharmaceutically compatible carrier (excipients) and/or wherein the composition is formed on an aqueous basis; and/or
wherein the composition is designed to be liquid or viscous, particularly viscous, preferably in a gelatinous form and/or wherein the composition exists in the form of a gel.

11. The composition according to one of the preceding claims,
wherein the composition additionally has at least one gel forming agent, particularly wherein the gel forming agent is chosen from the group of bentonites, silicic acids, polyacrylic acids, carbomers, polyvinylpyrrolidones, cellulose and cellulose derivatives, particularly esters and ethers of the cellulose, xanthane and their compounds; and/or
particularly wherein the composition contains the gel forming agent in a volume in the range of 0.01 to 20 wt.-%, particularly 0.1 to 10 wt.-%, preferably in the range of 0.5 to 5 wt.-%, more preferably in the range of 1 to 3 wt.-%, in relation to the composition; and/or
wherein the composition has at a temperature of 20 °C a dynamic viscosity in the range of 20 to 20,000 mPa·s, particularly in the range of 50 to 15,000 mPa·s, preferably in the range of 100 to 10,000 mPa·s, more preferably in the range of 250 to 8,000 mPa·s, particularly preferably in the range of 500 to 7,000 mPa·s, even more preferably in the range of 750 to 6,000 mPa·s; and/or
wherein the composition at a temperature of 20 °C has a density in the range of 0.5 to 2.0 g/cm³, particularly in the range of 0.7 to 1.8 g/cm³, preferably in the range of 1 to 1.7 g/cm³, more preferably in the range of 1.1 to 1.6 g/cm³; and/or wherein the composition contains at least one further active agent and/or ingredient, particularly chosen from the group of (mucous) membrane protection products, antiseptics, local anaesthetics, vitamins, trace elements, minerals, micronutrients and their combinations; and/or
wherein the composition contains at least one customary pharmaceutical additive and/or auxiliary agent, particularly chosen from the group of processing aids, stabilisers, emulsifiers, antioxidants, preservatives, humectants, pH suspending agents, pH buffer substances, thickening agents, antiseptics, colourants, buffer substances, aromatic substances, fragrances, diluting agents, binders, netting agents and/or preservatives, flavour-enhancing additives, flavours and sweeteners and their combinations.

12. The composition according to one of the preceding claims
for use in the field of medicine, pharmaceutics and food engineering and/or
for use in the prophylactic and/or curative treatment of illnesses and/or complaints of the gastric and/or intestinal tract, particularly stomach aches, bloating, flatulence, stomach and bowel cramps, nausea, heartburn, irritable stomach, gastritis, super acidity, oesophagitis, dyspeptic complaints, stomach ulcers, gastric discomfort, food intolerances and/or mucous membrane damage.

13. A container, particularly an application device, preferably in the form of a pump bottle or a sachet, particularly in the form of a pump bottle, containing a composition according to one of the preceding claims.

14. A pharmaceutical product, particularly for the prophylactic and/or curative treatment and/or for use in the prophylactic and/or curative treatment of illnesses and/or complaints of the gastric and/or intestinal tract, particularly stomach aches, bloating, flatulence, stomach and bowel cramps, nausea, heartburn, irritable stomach, gastritis, super acidity, oesophagitis, dyspeptic complaints, stomach ulcers, gastric discomfort, food intolerances and/or mucous membrane damage, containing a composition according to one of the preceding claims.

## Revendications

1. Composition, en particulier composition pharmaceutique, utilisée pour le traitement prophylactique et/ou curatif des maladies gastriques et/ou intestinales et/ou gastro-intestinales, la composition, contenant de manière combinée et dans des quantités respectivement efficaces, en particulier pharmaceutiquement efficaces,
(a) d'acide hyaluronique ou de ses sels ("composant (a)") dans une quantité relative située dans la plage allant de 0,2 à 5 % en poids rapporté à la composition, et
(b) une combinaison
d'extrait de fleur de camomille ("composant (b1)")
dans une quantité relative, rapporté à la composition, située dans la plage allant de 10 à 40 % en poids,
d'extrait de graine de carvi ("composant (b2)")
dans une quantité relative, rapporté à la composition, située dans la plage allant de 2 à 20 % en poids,
d'extrait de feuille de mélisse ("composant (b3)")
dans une quantité relative, rapporté à la composition, située dans la plage allant de 2 à 20 % en poids, et
d'extrait de feuille de menthe poivrée ("composant (b4)")
dans une quantité relative, rapporté à la composition, située dans la plage allant de 1 à 10 % en poids.

2. Composition selon la revendication 1, la composition présentant comme composant (b) une combinaison d'extrait de fleur de camomille ("composant (b1)"), d'extrait de graine de carvi ("composant (b2)"), d'extrait de feuille de mélisse ("composant (b3)"), l'extrait de feuille de menthe poivrée ("composant (b4)") et d'extrait de racine d'angélique ("composant (b5)").

3. Composition selon la revendication 1 ou 2,
la composition contenant le composant (a) dans une quantité relative située dans la plage allant de 0,3 à 2,5 % en poids, de manière davantage préférée de 0,5 à 1,5 % en poids, rapporté à la composition ; et/ou
la composition contenant le composant (a) dans une quantité absolue située dans la plage allant de 0,01 à 25 mg, en particulier dans la plage allant de 0,1 à 15 mg, de préférence dans la plage allant de 0,5 à 2,5 mg, de manière davantage préférée dans la plage allant de 0,6 à 2 mg, rapporté à une unité d'application de la composition ; et/ou
le composant (a) étant le sel sodique de l'acide hyaluronique ; et/ou
le composant (a), en particulier l'acide hyaluronique ou ses sels physiologiquement acceptables, présentant une masse moléculaire, en particulier une masse moléculaire moyenne en nombre, située dans la plage allant de 1 à 100 000 kDa (kilodaltons), en particulier dans la plage allant de 10 à 50 000 kDa, de préférence dans la plage allant de 100 à 10 000 kDa, de préférence dans la plage allant de 750 à 5 000 kDa, de manière particulièrement préférée dans la plage allant de 900 à 2 000 kDa.

4. Composition selon l'une quelconque des revendications précédentes,
la composition contenant un ou plusieurs extraits végétaux et/ou le composant (b) dans une quantité totale relative, rapporté à la composition, située dans la plage allant de 1 à 90 % en poids, en particulier dans la plage allant de 5 à 80 % en poids, de préférence dans la plage allant de 10 à 70 % en poids, de manière davantage préférée dans la plage allant de 20 à 65 % en poids, de manière particulièrement préférée dans la plage allant de 30 à 60 % en poids ; et/ou
la composition contenant un ou plusieurs extraits végétaux et/ou le composant (b) dans une quantité totale absolue située dans la plage allant de 0,1 à 1000 ml, en particulier dans la plage allant de 1 à 500 ml, de préférence dans la plage allant de 5 à 250 ml, de préférence dans la plage allant de 10 à 100 ml, de manière particulièrement préférée dans la plage allant de 20 à 80 ml, de manière davantage préférée dans la plage allant de 30 à 60 ml, rapporté à une unité d'application de la composition.

5. Composition selon l'une quelconque des revendications précédentes,
la composition contenant de l'extrait de fleur de camomille et/ou le composant (b1) dans une quantité relative, rapporté à la composition, située dans la plage allant de 15 à 30 % en poids ; et/ou
la composition contenant de l'extrait de fleur de camomille et/ou le composant (b1) dans une quantité absolue, rapporté à une unité d'application de la composition, située dans la plage allant de 0,1 à 70 ml, en particulier dans la plage allant de 1 à 60 ml, de préférence dans la plage allant de 5 à 50 ml, de manière davantage préférée dans la plage allant de 10 à 40 ml, de manière particulièrement préférée dans la plage allant de 15 à 30 ml ; et/ou
la composition contenant de l'extrait de graine de carvi et/ou le composant (b2) dans une quantité relative, rapporté à la composition, située dans la plage allant de 5 à 15 % en poids ; et/ou
la composition contenant de l'extrait de graine de carvi et/ou le composant (b2) dans une quantité absolue, rapporté à une unité d'application de la composition,
située dans la plage allant de 0,1 à 50 ml, en particulier dans la plage allant de 0,5 à 40 ml, de préférence dans la plage allant de 1 à 30 ml, de manière davantage préférée dans la plage allant de 2 à 20 ml, de manière particulièrement préférée dans la plage allant de 5 à 15 ml.

6. Composition selon l'une quelconque des revendications précédentes,
la composition contenant de l'extrait de feuille de mélisse et/ou le composant (b3) dans une quantité relative, rapporté à la composition, située dans la plage allant de 5 à 15 % en poids ; et/ou
la composition contenant de l'extrait de feuille de mélisse et/ou le composant (b3) dans une quantité absolue, rapporté à une unité d'application de la composition,
située dans la plage allant de 0,1 à 50 ml, en particulier dans la plage allant de 0,5 à 40 ml, de préférence dans la plage allant de 1 à 30 ml, de manière davantage préférée dans la plage allant de 2 à 20 ml, de manière particulièrement préférée dans la plage allant de 5 à 15 ml ; et/ou
la composition contenant de l'extrait de feuille de menthe poivrée et/ou le composant (b4) dans une quantité relative, rapporté à la composition, située dans la plage allant de 2,5 à 7,5 % en poids ; et/ou
la composition contenant de l'extrait de feuille de menthe poivrée et/ou le composant (b4) dans une quantité absolue, rapporté à une unité d'application de la composition, située dans la plage allant de 0,1 à 40 ml, en particulier dans la plage allant de 0,5 à 30 ml, de préférence dans la plage allant de 0,7 à 20 ml, de manière davantage préférée dans la plage allant de 1 à 10 ml, de manière particulièrement préférée dans la plage allant de 2,5 à 7,5 ml ; et/ou
la composition contenant de l'extrait de racine d'angélique et/ou le composant (b5) dans une quantité relative, rapporté à la composition, située dans la plage allant de 0,1 à 50 % en poids, en particulier dans la plage allant de 0,5 à 40 % en poids, de préférence dans la plage allant de 1 à 30 % en poids, de manière davantage préférée dans la plage allant de 2 à 20 % en poids, de manière particulièrement préférée dans la plage allant de 5 à 15 % en poids ; et/ou
la composition contenant de l'extrait de racine d'angélique et/ou le composant (b5) dans une quantité absolue, rapporté à une unité d'application de la composition, située dans la plage allant de 0,1 à 50 ml, en particulier dans la plage allant de 0,5 à 40 ml, de préférence dans la plage allant de 1 à 30 ml, de manière davantage préférée dans la plage de 2 à 20 ml, de manière particulièrement préférée dans la plage allant de 5 à 15 ml.

7. Composition, en particulier composition pharmaceutique, utilisée pour le traitement prophylactique et/ou curatif des maladies gastriques et/ou intestinales et/ou gastro-intestinales, en particulier composition selon l'une quelconque des revendications précédentes, la composition contenant, de manière combinée et dans des quantités respectivement efficaces, en particulier pharmaceutiquement efficaces,
(a) de l'acide hyaluronique ou ses sels ("composant (a)") dans une quantité relative située dans la plage allant de 0,2 à 5 % en poids rapporté à la composition ;
(b) une combinaison des extraits végétaux suivants, à savoir :
(b1) de l'extrait de fleur de camomille, en particulier dans une quantité relative, rapporté à la composition, située dans la plage allant de 10 à 40 % en poids, de manière particulièrement préférée dans la plage allant de 15 à 30 % en poids ;
(b2) de l'extrait de graine de carvi, en particulier dans une quantité relative, rapporté à la composition, située dans la plage allant de 2 à 20 % en poids, de manière particulièrement préférée dans la plage allant de 5 à 15 % en poids ;
(b3) de l'extrait de feuille de mélisse, en particulier dans une quantité relative, rapporté à la composition, située dans la plage allant de 2 à 20 % en poids, de manière particulièrement préférée dans la plage allant de 5 à 15 % en poids ;
(b4) de l'extrait de feuille de menthe poivrée, en particulier dans une quantité relative, rapporté à la composition, située dans la plage allant de 1 à 10 % en poids, de manière particulièrement préférée dans la plage allant de 2,5 à 7,5 % en poids ;
le cas échéant
(b5) de l'extrait de racine d'angélique , en particulier dans une quantité relative, rapporté à la composition, située dans la plage allant de 0,1 à 50 % en poids, en particulier dans la plage allant de 0,5 à 40 % en poids, de préférence dans la plage allant de 1 à 30 % en poids, de manière davantage préférée dans la plage allant de 2 à 20 % en poids, de manière particulièrement préférée dans la plage allant de 5 à 15 % en poids.

8. Composition selon l'une quelconque des revendications précédentes,
la composition contenant le composant (a) et le au moins un extrait végétal (b), en particulier les composants (b1) et/ou (b2) et/ou (b3) et/ou (b4) et/ou le cas échéant (b5) dans leur totalité, selon un rapport pondéral composant (a)/composant (b), en particulier selon un rapport pondéral composant (a)/somme des composants (b1) et/ou (b2) et/ou (b3) et/ou (b4) et/ou le cas échéant (b5), dans la plage allant de 10 : 1 à 1 : 1 000, en particulier dans la plage allant de 2 : 1 à 1 : 500, de préférence dans la plage allant de 1 : 1 à 1 : 100, de manière davantage préférée dans la plage allant de 1 : 5 à 1 : 75, de manière particulièrement préférée dans la plage allant de 1 : 10 à 1 : 50, et/ou
le au moins un extrait végétal, en particulier l'extrait de racine d'angélique, l'extrait de fleur de camomille, l'extrait de graine de carvi, l'extrait de feuille de mélisse et/ou l'extrait de feuille de menthe poivrée, présentant respectivement un rapport médicament/extrait situé dans la plage allant de 10 : 1 à 1 : 100, en particulier dans la plage allant de 5 : 1 à 1 : 50, de préférence dans la plage allant de 2 : 1 à 1 : 10, de manière davantage préférée dans la plage allant de 1 : 1 à 1 : 5 et/ou
le au moins un extrait végétal, en particulier l'extrait de racine d'angélique, l'extrait de fleur de camomille, l'extrait de graine de carvi, l'extrait de feuille de mélisse et/ou l'extrait de feuille de menthe poivrée, présentant respectivement un rapport médicament/extrait de 1 : 50, en particulier de 1 : 10, de préférence de 1 : 5, de manière davantage préférée de 1 : 4 ; et/ou
la composition présentant une teneur en alcool, en particulier une teneur en éthanol, inférieure à 5 % vol., en particulier inférieure à 3,0 % vol., de préférence inférieure à 2,0 % vol., de manière davantage préférée inférieure à 0,85 % vol., de manière particulièrement préférée inférieure à 0,5 vol., de manière particulièrement préférée inférieure à 0,25 % vol., de manière encore davantage préférée inférieure à 0,1 % vol., de manière préférée entre toutes d'environ 0 % vol., et/ou la composition étant réalisée au moins sensiblement sans alcool, en particulier au moins sensiblement sans éthanol.

9. Composition selon l'une quelconque des revendications précédentes,
le au moins un extrait végétal, en particulier l'extrait de racine d'angélique, l'extrait de fleur de camomille, l'extrait de graine de carvi, l'extrait de feuille de mélisse et/ou l'extrait de feuille de menthe poivrée, peut être obtenu (i) en préparant un extrait alcoolique, en particulier éthanolique, (ii) en évaporant ensuite le solvant alcoolique, en particulier l'éthanol et (iii) en recueillant au final l'extrait obtenu, en particulier l'extrait épais, dans un solvant approprié, en particulier organique, de préférence dans un solvant contenant du glycol, de manière davantage préférée dans du propylèneglycol ; et/ou
la composition étant au moins sensiblement dépourvue de composants inorganiques neutralisant (l'acide gastrique), en particulier de carbonates, d'hydrogénocarbonates et d'hydroxydes, en particulier d'hydroxyde d'aluminium et/ou la composition étant sensiblement dépourvue d'antiacides ; et/ou
la composition présentant au moins un système tampon de pH, le système tampon étant en particulier choisi parmi les systèmes tampons physiologiquement acceptables, de préférence le tampon acide citrique/citrate ou le tampon acide acétique/acétate, de préférence le tampon acide citrique/citrate ; et/ou
la composition présentant une valeur de pH physiologiquement acceptable, en particulier une valeur de pH située dans la plage allant de 4 à 9, en particulier dans la plage allant de 5 à 8, de préférence dans la plage allant de 5,5 à 6.

10. Composition selon l'une quelconque des revendications précédentes,
la composition contenant au moins un conservateur pharmaceutiquement acceptable ;
le conservateur étant en particulier choisi parmi les phénols et leurs dérivés, en particulier les alcools benzyliques, le chlorobutanol et/ou le propylèneglycol, les alcools aliphatiques et aromatiques, en particulier les crésols, les parabènes et/ou les chlorocrésols, les composés d'ammonium quaternaire, en particulier le chlorure de benzalkonium et/ou le chlorure de cétylpyridinium, et les acides carboxyliques et leurs sels, en particulier l'acide sorbique et/ou l'acide benzoïque et/ou leurs sels, de manière particulièrement préférée l'acide sorbique et ses sels (sorbates), en particulier un sorbate alcalin comme le sorbate de potassium ;
et/ou
en particulier la composition contenant le au moins un conservateur pharmaceutiquement acceptable dans une quantité relative située dans la plage allant de 0,001 à 3 % en poids, en particulier allant de 0,01 à 2 % en poids, de préférence dans la plage allant de 0,05 à 1 % en poids, de manière davantage préférée dans la plage allant de 0,1 à 0,5 % en poids, rapporté à la composition ;
et/ou
en particulier le conservateur étant l'acide sorbique et/ou un sel de l'acide sorbique, en particulier le sel sodique ou potassique de l'acide sorbique ; et/ou
la composition contenant de l'eau, en particulier de l'eau purifiée, dans une quantité située dans la plage allant de 1 à 70 % en poids, en particulier dans la plage allant de 5 à 50 % en poids, de manière davantage préférée dans la plage allant de 10 à 40 % en poids, de manière particulièrement préférée dans la plage allant de 15 à 30 % en poids, rapporté à la composition, et/ou la composition contenant de l'eau en tant que véhicule (excipient) pharmaceutiquement acceptable et/ou la composition étant conçue sur une base aqueuse ; et/ou
la composition étant conçue sous forme fluide ou visqueuse, en particulier visqueuse, de préférence sous forme de gel, et/ou la composition étant disponible sous forme de gel.

11. Composition selon l'une quelconque des revendications précédentes,
la composition présentant en outre au moins un agent gélifiant,
en particulier l'agent gélifiant étant choisi dans le groupe des bentonites, des acides siliciques, des acides polyacryliques, des carbomères, des polyvinylpyrrolidones, de la cellulose et des dérivés de cellulose, en particulier des esters et des éthers de cellulose, des xanthanes et leurs mélanges ; et/ou
en particulier la composition présentant l'agent gélifiant dans une quantité située dans la plage allant de 0,01 à 20 % en poids, en particulier dans la plage allant de 0,1 à 10 % en poids, de préférence dans la plage allant de 0,5 à 5 % en poids, de manière davantage préférée dans la plage allant de 1 à 3 % en poids, rapporté à la composition ; et/ou
la composition présentant, à une température de 20 °C, une viscosité dynamique située dans la plage allant de 20 à 20 000 mPa·s, en particulier dans la plage allant de 50 à 15 000 mPa·s, de préférence dans la plage allant de 100 à 10 000 mPa·s, de manière davantage préférée dans la plage allant de 250 à 8 000 mPa·s, de manière particulièrement préférée dans la plage allant de 500 à 7 000 mPa·s, de manière encore davantage préférée dans la plage allant de 750 à 6 000 mPa·s ; et/ou
la composition présentant, à une température de 20 °C, une densité située dans la plage allant de 0,5 à 2,0 g/cm³, en particulier dans la plage allant de 0,7 à 1,8 g/cm³, de préférence dans la plage allant de 1 à 1,7 g/cm³, de manière davantage préférée dans la plage allant de 1,1 à 1,6 g/cm³ ; et/ou
la composition contenant au moins un autre agent actif et/ou ingrédient choisi en particulier dans le groupe des agents protecteurs pour la peau (et les muqueuses), les antiseptiques, les anesthésiques locaux, les vitamines, les oligo-éléments, les minéraux, les micro-nutriments et leurs combinaisons ; et/ou
la composition contenant au moins un additif et/ou un auxiliaire pharmaceutique habituel, choisi(s) en particulier dans le groupe des auxiliaires de préparation, des stabilisateurs, des émulsifiants, des antioxydants, des conservateurs, des agents humectants, des ajusteurs de pH, des substances tampons de pH, des épaississants, des antiseptiques, des colorants, des tampons, des substances odorantes, des parfums, des diluants, des liants, des agents de réticulation et/ou des conservateurs, des agents de sapidité, des arômes et des édulcorants et leurs combinaisons.

12. Composition selon l'une quelconque des revendications précédentes,
destinée à être utilisée dans le domaine de la médecine, de la pharmacie et de l'industrie alimentaire et/ou
destinée à être utilisée pour le traitement prophylactique et/ou curatif des maladies et/ou des troubles gastriques et/ou intestinaux, en particulier des douleurs gastriques, des ballonnements, des flatulences, des crampes de l'estomac et de l'intestin, des nausées, des brûlures d'estomac, de la dyspepsie fonctionnelle, de la gastrite, de l'hyperacidité, de l'oesophagite, des douleurs dyspeptiques, des ulcères de l'estomac, des lourdeurs d'estomac, des intolérances alimentaires et/ou des lésions des muqueuses.

13. Récipient, en particulier dispositif d'application, de préférence sous la forme d'un flacon-pompe ou d'un sachet-dose, en particulier sous la forme d'un flacon-pompe, contenant une composition selon l'une quelconque des revendications précédentes.

14. Médicament, en particulier pour le traitement prophylactique et/ou curatif et/ou destiné à être utilisé pour le traitement prophylactique et/ou curatif des maladies et/ou des troubles gastriques et/ou intestinaux, en particulier des douleurs gastriques, des ballonnements, des flatulences, des crampes de l'estomac et de l'intestin, des nausées, des brûlures d'estomac, de la dyspepsie fonctionnelle, de la gastrite, de l'hyperacidité, de l'oesophagite, des douleurs dyspeptiques, des ulcères de l'estomac, des lourdeurs d'estomac, des intolérances alimentaires et/ou des lésions des muqueuses, contenant une composition selon l'une quelconque des revendications précédentes.
